# EUROPEAN PATENT SPECIFICATION

(11) **EP 3 065 762 B1**
(45) Date of publication and mention of the grant of the patent: **10.10.2018**
(21) Application number: 14802543.0
(22) Date of filing: 04.11.2014
(51) Int. Cl.: A61P 7/08, A61P 7/06

(54) **METHODS OF REDUCING DOSES OF ERYTHROPOIETIN STIMULATING AGENTS IN HYPORESPONSIVE PATIENTS**
VERFAHREN ZUR REDUZIERUNG ERYTHROPOIETIN-STIMULIERENDER MITTEL IN HYPORESPONSIVEN PATIENTEN
MÉTHODES DESTINÉES À RÉDUIRE LES DOSES D'AGENTS STIMULANT L'ÉRYTHROPOÏÉTINE CHEZ LES PATIENTS HYPORÉACTIFS

(30) Priority: 05.11.2013 US 201361900387 P
(43) Date of publication of application: 14.09.2016
(73) Proprietor: Rockwell Medical Inc., Wixom, MI 48393 (US)
(72) Inventor: GUPTA, Ajay, Cerritos, CA 90703 (US); PRATT, Raymond, Baltimore, MD 21202 (US); LIN, Vivian H., Ann Arbor, MI 48105 (US); GUSS, Carrie, Northville, MI 48168 (US)
(74) Representative: Grünecker Patent- und Rechtsanwälte PartG mbB
(86) International application number: PCT/US2014/063919
(87) International publication number: WO 2015/069656

(56) References cited:
- WO-A1-2014/121155
- WO-A2-2012/094598
- US-A1- 2007 012 622
- Michael Rice: "Rockwell Medical Schedules 3rd Quarter 2013 Investor Conference Call", Rockwell Medical Technologies, Inc. , 29 October 2013 (2013-10-29), XP002733823, Retrieved from the Internet: URL:http://files.shareholder.com/downloads /RMTI/3728797109x0x700920/ac8e1a85-500c-42 f2-970b-a48a2874bca6/RMTI_News_2013_10_29_ General.pdf [retrieved on 2014-12-15]
- Michael Rice: "Rockwell Medical Announces Presentation Schedule for Triferic(TM) Clinical Posters at the American Society of Nephrology (ASN) Meeting November 2013", Rockwell Medical Technologies, Inc. , 24 October 2013 (2013-10-24), XP002733824, Retrieved from the Internet: URL:http://files.shareholder.com/downloads /RMTI/3728797109x0x698351/6a3b7af7-153c-4f e8-8080-acb3a887f557/RMTI_News_2013_10_21_ General.pdf [retrieved on 2014-12-15]
- Michael Rice: "Rockwell Medical Announces Clean Safety Results From Triferic(TM) Short-Term Safety Study", Rockwell Medical Technologies, Inc. , 21 October 2013 (2013-10-21), XP002733825, Retrieved from the Internet: URL:http://files.shareholder.com/downloads /RMTI/3728797109x0x698351/6a3b7af7-153c-4f e8-8080-acb3a887f557/RMTI_News_2013_10_21_ General.pdf [retrieved on 2014-12-15]
- Jody A. Charnow, Editor: "Advantages Cited with Novel Iron Drug", , 11 April 2013 (2013-04-11), XP002733826, Retrieved from the Internet: URL:http://www.renalandurologynews.com/adv antages-cited-with-novel-iron-drug/article /288463/ [retrieved on 2014-12-15]
- A BESARAB ET AL: "Optimization of epoetin therapy with intravenous iron therapy in hemodialysis patients", JOURNAL OF THE AMERICAN SOCIETY OF NEPHROLOGY., vol. 11, no. 3, 1 March 2000 (2000-03-01), pages 530-538, XP055158536, US ISSN: 1046-6673
- DAVID W JOHNSON ET AL: "Erythropoiesis-stimulating agent hyporesponsiveness (Review Article)", NEPHROLOGY, vol. 12, no. 4, 1 August 2007 (2007-08-01) , pages 321-330, XP055158530, ISSN: 1320-5358, DOI: 10.1111/j.1440-1797.2007.00810.x
- GUPTA A ET AL: "Treatment of iron deficiency anemia: Are monomeric iron compounds suitable for parenteral administration?", JOURNAL OF LABORATORY AND CLINICAL MEDICINE, ORLANDO, FLA : ELSEVIER, 1915-2006ST. LOUIS, MO : MOSBY, US, vol. 136, no. 5, 1 November 2000 (2000-11-01), pages 371-378, XP002667367, ISSN: 0022-2143, DOI: 10.1067/MLC.2000.110368 [retrieved on 2002-05-16]
- Andrew I. McDonald et Al.: "Triferic Data Presented at 2013 American Society of Nephrology Meeting (ASN)Strong Support for Upcoming NDA Submission & Impressive ESA Sparing Data", LIFESCI ADVISORS - Company Note , 14 November 2013 (2013-11-14), pages 1-8, XP002733827, Retrieved from the Internet: URL:http://www.lifesciadvisors.com/clienti nfo/rockwell/Rockwell__ASN_Kidney_Week_11- 14-2013__clientinfo.pdf [retrieved on 2014-12-15]
- RAYMOND PRATT, VIVIAN LIN, CARRIE GUSS, GUPTA AJAY. ROCKWELLMEDICAL, WIXOM, MICHIGAN, UNITED STATES: "Triferic (Soluble Ferric Pyrophosphate) Administered ViaDialysate Maintains Hemoglobin and Reduces ESA and IVIron Requirements in CKD-HD", HEMODIALYSIS INTERNATIONAL, vol. 18, 8 January 2014 (2014-01-08), pages 234-235, XP002733828, ISSN: 1542-4758

## Description

This application claims priority to U.S. provisional patent application no. 61/900,387, filed November 5, 2013.

### FIELD OF INVENTION

Disclosed are methods of treating a dialysis patient that is hyporesponsive to erythropoietin stimulating agents (ESA) comprising administering soluble ferric pyrophosphate (SFP), and administering a dose of ESA that is significantly reduced than the dose of ESA required by a dialysis patient that is hyporesponsive to ESA that has not received SFP.

### BACKGROUND

Anemia is a common clinical feature of disease states characterized by concomitant inflammation such as cancer, autoimmune diseases, chronic infections and chronic kidney disease (CKD). The anemia in CKD patients is multifactorial in origin (Babitt & Lin, J. Am. Soc. Nephrol. 23: 1631, 1634, 2012). The anemia of CKD was previously largely attributed to impaired production of erythropoietin by the diseased kidneys. Treatment with recombinant human erythropoietin or other erythropoiesis stimulating agents (ESA) improves or prevents anemia partially but may be associated with adverse effects including worsening hypertension, seizures, and dialysis access clotting. In addition, ESAs may lead to increased risk of death, adverse cardiovascular events and strokes when administered to achieve hemoglobin (Hgb) levels > 11 g/dL.

Hyporesponsiveness to ESAs or resistance to ESA therapy is a common finding of grave significance whether it is manifested by persistent, below-target Hgb levels despite substantial ESA doses or by within-target Hgb levels attained only at very high ESA doses. Given the disproportionate burden of morbidity and mortality that the ESA hyporesponsive patient population bears and the ESA expense that hyporesponsiveness engenders, hyporesponsiveness to ESAs deserves more scrutiny than it has received.

There are a number of causes of hyporesponse to ESA therapy such as acute blood loss such as from surgery, vascular access interventions and gastrointestinal bleeding, acute infection/inflammation, aluminum toxicity, chronic blood loss such as from frequent clotting of dialyzer, excessive port-dialysis bleeding, frequent phlebotomy, chronic infection/inflammation such as systemic lupus erythematosus, failed kidney transplant, catheter associated inflammation, diabetes, hyperparathyroidism, iron deficiency, medications, nonadherance to ESA or iron therapy, pure red blood cell aplasia such as production of antibodies that neutralize ESA and endogenous erythropoietin, uremia/suboptimal dialysis, vitamin and/or mineral deficiency and HIV.

Iron deficiency is a major contributor to anemia in CKD patients. Absolute iron deficiency with depletion of iron stores can be corrected by intravenous administration of iron. However, the efficacy of intravenous iron in the setting of anemia of inflammation as in patients with CKD is impaired, since intravenously administered iron-carbohydrate complexes get trapped in the reticulo-endothelial system (RES) by the action of hepcidin, a peptide released from the liver cells in states of inflammation (Babitt & Lin, J. Am. Soc. Nephrol. 23: 1631, 1634, 2012). This results in reticulo-endothelial block and thereby a state of functional iron deficiency.

Therefore, there is a need for methods of treating anemia in dialysis patients and reducing the ESA dosages administered to these patients, in particular those patients that are hyporesponsive to ESA.

### SUMMARY OF INVENTION

The invention is as defined in the claims. The administration of soluble ferric pyrophosphate (SFP) can overcome both absolute and functional iron deficiencies in CKD-HD patients. SFP-iron directly binds to apo-transferrin, thereby delivering SFP-iron to bone marrow directly, bypassing the RES (Gupta et al. J Am Soc Nephrol 2010; 21 (Renal Week 2010 Abstract Supplement): 429A, 2010). SFP has been shown herein to significantly reduce the amount of ESA needed to treat ESA-hyporesponsive dialysis patients. The invention provides for method of treating dialysis patients that are hyporesponsive to ESA therapy and involve reducing the dose of ESA by at least 50% compared to ESA hyporesponsive dialysis patients that have not received SFP.

"Hyporesponsive or resistance to ESA therapy" is defined as a condition in which there is a significant increase in the ESA dose requirement to maintain an Hgb level within a specified Hgb range or a significant decrease in Hgb concentration at a constant ESA dose or a failure to raise the Hgb level to the target range despite the ESA dose equivalent to erythropoietin greater than 150 IU/kg/week or 0.75 mg/kg/week of darbepoeitn-alpha or continued need for such high dose of ESA to maintain the target Hgb level. For example, approximately 5-10% of patients with CDK demonstrate hyporesponsiveness to ESA, defined as a continued need for greater than 300 IU/kg per week erythropoietin or 1.5 mug/kg per week darbepoetin administered by the subcutaneous route (Johnston et al., Nephrology. 2007 Aug;12(4):321-30). In the clinical study described in Examples 1 and 2, about 20% of study patients, the relatively ESA hyporesponsive group, was receiving greater than 13,000 Unit of ESA per week at the start of the study (Note: Patients with ESA doses exceeding 45,000 units per week were excluded from the study).

Disclosed are methods of treating dialysis patients that are hyporesponsive to ESA comprising: (a) administering to the patient a SFP composition, and (b) administering to the patient a dose of ESA that is at least 50% less than the dose of ESA required by dialysis patients that are hyporesponsive to ESA and that have not received SFP.

Disclosed are methods of reducing the dose of ESA in dialysis patients that are hyporesponsive to ESA comprising (a) administering to the patient a SFP composition, and (b) after SFP administration, administering to the patient a dose of ESA that is at least 50% less than the dose of ESA required by dialysis patients that are hyporesponsive to ESA and have not received SFP.

Disclosed are methods of treating or preventing iron deficiency in a dialysis patent that is hyporesponsive to ESA comprising (a) administering to the patient a SFP composition and (b) administering to the patient a dose of ESA that is at least 50% less than the dose of ESA required by dialysis patients that are hyporesponsive to ESA and have not received SFP.

In any of the methods, the dose of SFP is administered via a parenteral route selected from the group consisting of intramuscular, subcutaneous, intravenous, intradermal, transdermal, transbuccal, oral, sublingual, intra-peritoneal, via dialysate, in conjunction with hemodialysis or in conjunction with peritoneal dialysis. The dose of SFP may be a dose that effectively treats or prevents iron deficiency or anemia such as a dose of SFP required to treat dialysis patients such as to achieve or maintain target hemoglobin level. Optionally, any of these methods, uses or compositions for use described herein may further comprise the step of reducing the dose of intravenously administered iron.

"Oral delivery" refers to administering a therapeutic agent such as a dose of SFP by mouth leading to gastrointestinal absorption. "Parenteral delivery" refers to administering a therapeutic agent, such as a dose of SFP, by injection or infusion, such as via intravenous, subcutaneous, intramuscular, intradermal, transdermal, transbuccal, sublingual or intra-peritoneal route. Parenteral delivery also includes administration via hemodialysis when added to (or in conjunction with) hemodialysis solutions, via peritoneal dialysis when added to (or in conjunction with) peritoneal dialysis solutions, or in conjunction with parenteral nutrition admixtures when added to parenteral nutrition admixture.

Disclosed are compositions of SFP for use in treating a dialysis patient that is hyporesponsive to erythropoietin stimulating agent (ESA). Treatment of this subpopulation of patients results in unexpectedly better ESA-sparing results, compared to the general dialysis population.

Disclosed are compositions of SFP for use in treating a dialysis patient that is hyporesponsive to erythropoietin stimulating agent (ESA) wherein the dose of SFP is effective to reduce the dose of ESA administered to the patient by least 50% less than the dose of ESA required by dialysis patients that are hyporesponsive to ESA and that have not received SFP.

Disclosed are compositions of SFP for use in reducing the dose of ESA in dialysis patients that are hyporesponsive to ESA wherein the dose of SFP is effective to reduce the dose of ESA administered to the patient by at least 50% less than the dose of ESA required by dialysis patients that are hyporesponsive to ESA and have not received SFP.

Disclosed are compositions of SFP for use in treating or preventing iron deficiency in a dialysis patient that is hyporesponsive to ESA wherein the dose of ESA is effective to reduce the dose of ESA that is administered to the patient by at least 50% less than the dose of ESA required by dialysis patients that are hyporesponsive to ESA and have not received SFP.

Disclosed is the use of SFP for the preparation of a medicament for the treatment of a dialysis patient that is hyporesponsive to ESA. In some embodiments, the SFP is at a dose that is effective to reduce the dose of ESA administered to the patient by least 50% less than the dose of ESA required by dialysis patients that are hyporesponsive to ESA and that have not received SFP.

Disclosed is the use of SFP for the preparation of a medicament for the reduction of the dose of ESA in dialysis patients that are hyporesponsive to ESA wherein SFP is at a dose that is effective to reduce the dose of ESA administered to the patient by at least 50% less than the dose of ESA required by dialysis patients that are hyporesponsive to ESA and have not received SFP.

Disclosed is the use of SFP for the preparation of a medicament for the treatment or prevention of iron deficiency in a dialysis patient that is hyporesponsive to ESA wherein SFP is at a dose that is effective to reduce the dose of ESA that is administered to the patient by at least 50% less than the dose of ESA required by dialysis patients that are hyporesponsive to ESA and have not received SFP.

In any of the preceding methods, uses or compositions, the dose of SFP is administered in conjunction with other drugs such as heparin or parenteral nutrition admixtures or dialysis solutions.

In any of the preceding methods, uses or compositions, the dose of ESA administered to the dialysis patient that is hyporesponsive to ESA after SFP administration is at least 50% less than the dose of ESA required by a dialysis patients that are hyporesponsive to ESA and have not received SFP, the dose is least 55% less than the dose of ESA required by a dialysis patients that are hyporesponsive to ESA and have not received SFP, the dose is at least 60% less than the dose of ESA required by a dialysis patients that are hyporesponsive to ESA and have not received SFP, the dose is at least 65% less than the dose of ESA required by a dialysis patients that are hyporesponsive to ESA and have not received SFP, the dose is at least 70% less than the dose of ESA required by a dialysis patients that are hyporesponsive to ESA and have not received SFP, the dose is at least 75% less than the dose of ESA required by a dialysis patients that are hyporesponsive to ESA and have not received SFP, the dose is at least 80% less than the dose of ESA required by a dialysis patients that are hyporesponsive to ESA and have not received SFP, the dose is at least 85% less than the dose of ESA required by a dialysis patients that are hyporesponsive to ESA and have not received SFP, the dose is at least 90% less than the dose of ESA required by a dialysis patients that are hyporesponsive to ESA and have not received SFP, the dose is or at least 95% less than the dose of ESA required by a dialysis patients that are hyporesponsive to ESA and have not received SFP.

In addition, in any of the preceding methods, use or compositions, the dose of ESA administered to the dialysis patient that is hyporeponsive to ESA after SFP administration ranges from about 50% to about 95% less than the dose of ESA required by a dialysis patients that are hyporesponsive to ESA and have not received SFP, or ranges from about 50% to about 90% less than the dose of ESA required by a dialysis patients that are hyporesponsive to ESA and have not received SFP, or ranges from about 50% to about 85% less than the dose of ESA required by a dialysis patients that are hyporesponsive to ESA and have not received SFP, or ranges from about 50% to about 80% less than the dose of ESA required by a dialysis patients that are hyporesponsive to ESA and have not received SFP, or ranges from about 50% to about 75% less than the dose of ESA required by a dialysis patients that are hyporesponsive to ESA and have not received SFP, or ranges from 50% to about 70% less than the dose of ESA required by a dialysis patients that are hyporesponsive to ESA and have not received SFP, or ranges from about 50% to about 65% less than the dose of ESA required by a dialysis patients that are hyporesponsive to ESA and have not received SFP, or ranges from 50% to about 60% less than the dose of ESA required by a dialysis patients that are hyporesponsive to ESA and have not received SFP, or ranges from about 60% to about 95% less than the dose of ESA required by a dialysis patients that are hyporesponsive to ESA and have not received SFP, or ranges from about 60% to about 90% less than the dose of ESA required by a dialysis patients that are hyporesponsive to ESA and have not received SFP, or ranges from about 60% to about 85% less than the dose of ESA required by a dialysis patients that are hyporesponsive to ESA and have not received SFP, or ranges from about 60% to about 80% less than the dose of ESA required by a dialysis patients that are hyporesponsive to ESA and have not received SFP, or ranges from about 60% to about 75% less than the dose of ESA required by a dialysis patients that are hyporesponsive to ESA and have not received SFP, or ranges from 60% to about 70% less than the dose of ESA required by a dialysis patients that are hyporesponsive to ESA and have not received SFP, or ranges from about 70% to about 95% less than the dose of ESA required by a dialysis patients that are hyporesponsive to ESA and have not received SFP, or ranges from about 70% to about 90% less than the dose of ESA required by a dialysis patients that are hyporesponsive to ESA and have not received SFP, or ranges from about 70% to about 85% less than the dose of ESA required by a dialysis patients that are hyporesponsive to ESA and have not received SFP, or ranges from about 70% to about 80% less than the dose of ESA required by a dialysis patients that are hyporesponsive to ESA and have not received SFP, from about 75% to about 95% less than the dose of ESA required by a dialysis patients that are hyporesponsive to ESA and have not received SFP, or ranges from about 75% to 90% less than the dose of ESA required by a dialysis patients that are hyporesponsive to ESA and have not received SFP, or ranges from about 75% to about 85% less than the dose of ESA required by a dialysis patients that are hyporesponsive to ESA and have not received SFP, or ranges from about 75% to about 80% less than the dose of ESA required by a dialysis patients that are hyporesponsive to ESA and have not received SFP, from about 80% to about 95% less than the dose of ESA required by a dialysis patients that are hyporesponsive to ESA and have not received SFP, or ranges from about 80% to about 90% less than the dose of ESA required by a dialysis patients that are hyporesponsive to ESA and have not received SFP, or ranges from about 80% to about 85% less than the dose of ESA required by a dialysis patients that are hyporesponsive to ESA and have not received SFP, or ranges from about 90% to about 95% less than the dose of ESA required by a dialysis patients that are hyporesponsive to ESA and have not received SFP, or ranges from about 90% to 98% less than the dose of ESA required by a dialysis patients that are hyporesponsive to ESA and have not received SFP.

Disclosed is also treating and/or preventing iron deficiency in dialysis patients with or without anemia, preferably dialysis patients that are hyporesponsive to ESA. These methods comprise administering a therapeutically effective dose of SFP. In patients with iron deficiency anemia, the therapeutically effective dose of SFP will increase or stabilize markers of iron status such as serum iron, transferrin saturation, reticulocyte hemoglobin, serum ferritin, reticulocyte count, and whole blood hemoglobin while reducing or eliminating the need for ESAs if the patient is a candidate for ESA administration. Furthermore, the therapeutically effective dose will reduce or eliminate the need for transfusion of whole blood or packed red blood cell or blood substitutes. In non-anemic patients with iron deficiency, as in patients with iron deficiency anemia, the therapeutically effective dose of SFP will reduce fatigue, increase physical and cognitive ability, improve exercise tolerance. If an iron deficient patient, with or without anemia, suffers from restless leg syndrome (RLS) the therapeutically effective dose of SFP will reduce or abolish the clinical manifestations of RLS.

Dialysis patients are at risk for suffering from iron deficiency and accordingly are at risk of developing anemia. Anemic subjects have reduced Hgb levels and the methods, compositions and uses of the invention may administer a dose of SFP effective to increases Hgb levels in the dialysis patient who is either suffering from anemia or treated to prevent anemia. For example, the dose of SFP administered increases Hgb level to that which is sufficient to adequately oxygenate the subject's tissues or provides improved oxygenation of the anemic subject's tissues. Preferably, the dose of SFP administered increases or maintains the Hgb level of the subject at 9-10 g/dL or greater thereby reducing the need for blood transfusions, reducing fatigue, improving physical and cognitive functioning, improving cardiovascular function, improving exercise tolerance and enhancing quality of life. For example, Hgb levels are increased to or maintained at a target level ranging from 9 to 10 g/dL, at a target level ranging from 9 g/dL to 11 g/dL, at a target level ranging from 9 g/dL to 12 g/dL, at a target level ranging from 9 g/dL to 14 g/dL, at a target level ranging from 10 g/dL to 14 g/dL, or at a target level ranging from 12 g/dL to 14 g/dL.

In any of the preceding methods, uses or compositions, the dose of SFP administered may be effective to increase or maintain Hgb at a target level of at least about 9 g/dL, of at least about 10 g/dL, of at least about 11 g/dL, of at least about 12 g/dL, of at least about 13 g/dL of at least about 14 g/dL, of about 9-11 g/dL, of about 9-12 g/dL or at about 9-14 g/dL.

In an aspect of the invention, the dose of SFP for any of the preceding methods, compositions or uses is administered via hemodialysate at a iron dose ranging from 90 µg/L dialysate to 150 µg/L dialysate, or at a dose ranging from 90 µg/L dialysate to 140 µg/L dialysate, or at a dose ranging from 90 µg/L dialysate to 130 µg/L dialysate, or at a dose ranging from 90 µg/L dialysate to 120 µg/L dialysate, or at a dose ranging from 90 µg/L dialysate to 110 µg/L dialysate, or at a dose ranging from 90 µg/L dialysate to 105 µg/L dialysate, or at a dose ranging from 105 µg/L dialysate to 115 µg/L dialysate, or at a dose ranging from 105 µg/L dialysate to 110 µg/L dialysate, or at a dose ranging from 105 µg/L dialysate to 120 µg/L dialysate, or at a dose ranging from 105 µg/L dialysate to 130 µg/L dialysate, or at a dose ranging from 105 µg/L dialysate to 140 µg/L dialysate, or at a dose ranging from 105 µg/L dialysate to 150 µg/L dialysate, or at a dose ranging from 110 µg/L dialysate to 150 µg/L dialysate, or at a dose ranging from 110 µg/L dialysate to 140 µg/L dialysate, or at a dose ranging from 110 µg/L dialysate to 130 µg/L dialysate, or at a dose ranging from 110 µg/L dialysate to 120 µg/L dialysate, or at a dose ranging from 110 µg/L dialysate to 115 µg/L dialysate, or at a dose ranging from 112 µg/L dialysate to 150 µg/L dialysate, or at a dose ranging from 112 µg/L dialysate to 140 µg/L dialysate, or at a dose ranging from 112 µg/L dialysate to 130 µg/L dialysate, or at a dose ranging from 112 µg/L dialysate to 120 µg/L dialysate, or at a dose ranging from 112 µg/L dialysate to 118 µg/L dialysate, or at a dose ranging from 112 µg/L dialysate to 115 µg/L dialysate, or at a dose ranging from 115 µg/L dialysate to 150 µg/L dialysate, or at a dose ranging from 115 µg/L dialysate to 140 µg/L dialysate, or at a dose ranging from 115 µg/L dialysate to 130 µg/L dialysate, or at a dose ranging from 115 µg/L dialysate to 120 µg/L dialysate, or at a dose ranging from 120 µg/L dialysate to 150 µg/L dialysate, or at a dose ranging from 120 µg/L dialysate to 140 µg/L dialysate, or at a dose ranging from 120 µg/L dialysate to 130 µg/L dialysate, or at a dose ranging from 120 µg/L dialysate to 125 µg/L dialysate, or at a dose ranging from 130 µg/L dialysate to 150 µg/L dialysate, or at a dose ranging from 130 µg/L dialysate to 140 µg/L dialysate, or at a dose ranging from 140 µg/L dialysate to 150 µg/L dialysate.

In an exemplary aspect of the invention, the dose of SFP is administered for any of the preceding methods, compostions or uses at a dose of 110 µg or 2 µmoles SFP- iron per liter of hemodialysate. Disclosed are methods wherein the dose of SFP iron is administered via dialysate at a dose of about 105 µg Fe/L dialysate, about 106 µg Fe/L dialysate, about 107 µg Fe/L dialysate, about 108 µg Fe/L dialysate, about 109 µg Fe/L dialysate, about 110 µg Fe/L dialysate, about 111 µg Fe/L dialysate or about 112 µg Fe/L dialysate.

As further examples, the dose of SFP administered to a dialysis patient that is hyporesponsive to ESA is about 105 µg Fe/L dialysate to about 115 µg Fe/L dialysate, and, after SFP administration, the ESA dose administered to the patient is about 50%-85% less than administered to a similar patient that has not received SFP.

In one aspect, the dose of SFP for any of the preceding methods, compositions or uses is administered via infusion at a dose ranging from 2.4 mg to 48 mg iron per day at a rate of 0.1 to 2 mg iron per hour. In another aspect, the dose of SFP is administered via intravenous injection at a dose ranging from 2.4 mg to 48 mg iron per day at a rate of 0.1 to 2 mg iron per hour. In addition, disclosed are any of the preceding methods wherein, the dose of SFP is administered into the circulation at a dose ranging from 2.4 mg to 48 mg iron per day at a rate of 0.1 to 2 mg iron per hour. For any of these methods, compositions or uses, the dose administered to the subject is based on the bioavailability of SFP using the specific route of administration.

Additional exemplary dose ranges for administering SFP-iron via infusion, intravenous injection or delivery into the circulation include a dose ranging from 5 mg to 48 mg per day at a rate of 0.1 to 2 mg per hour, or at a dose ranging from 10 mg to 48 mg per day at a rate of .01 to 2 mg per hour, or at a dose ranging from 20 mg to 48 mg per day at a rate of .01 to 2 mg per hour, or at a dose ranging from 30 mg to 48 mg per day at a rate of .01 to 2 mg per hour, or at a dose ranging from 40 mg to 48 mg per day at a rate of .01 to 2 mg per hour, or a dose ranging from 2.4 mg to 48 mg per day at a rate of 1 to 2 mg per hour, or a dose ranging from 5 mg to 48 mg per day at a rate of 1 to 2 mg per hour, or at a dose ranging from 10 mg to 48 mg per day at a rate of 1 to 2 mg per hour, or at a dose ranging from 20 mg to 48 mg per day at a rate of 1 to 2 mg per hour, or at a dose ranging from 30 mg to 48 mg per day at a rate of 1 to 2 mg per hour, or at a dose ranging from 40 mg to 48 mg per day at a rate of 1 to 2 mg per hour, or a dose ranging from 2.4 mg to 48 mg per day at a rate of 0.5 to 1 mg per hour, or a dose ranging from 5 mg to 48 mg per day at a rate of 0.5 to 1 mg per hour, or at a dose ranging from 10 mg to 48 mg per day at a rate of 0.5 to 1 mg per hour, or at a dose ranging from 20 mg to 48 mg per day at a rate of 0.5 to 1 mg per hour, or at a dose ranging from 30 mg to 48 mg per day at a rate of .0.5 to 1 mg per hour, or at a dose ranging from 40 mg to 48 mg per day at a rate of 0.5 to 1 mg per hour, a dose ranging from 2.4 mg to 40 mg per day at a rate of 0.1 to 2 mg per hour, or a dose ranging from 5 mg to 40 mg per day at a rate of 0.1 to 2 mg per hour, or at a dose ranging from 10 mg to 40 mg per day at a rate of .01 to 2 mg per hour, or at a dose ranging from 20 mg to 40 mg per day at a rate of .01 to 2 mg per hour, or at a dose ranging from 30 mg to 40 mg per day at a rate of .01 to 2 mg per hour, or at a dose ranging from 40 mg to 40 mg per day at a rate of .01 to 2 mg per hour, or a dose ranging from 2.4 mg to 40 mg per day at a rate of 1 to 2 mg per hour, or a dose ranging from 5 mg to 40 mg per day at a rate of 1 to 2 mg per hour, or at a dose ranging from 10 mg to 40 mg per day at a rate of 1 to 2 mg per hour, or at a dose ranging from 20 mg to 40 mg per day at a rate of 1 to 2 mg per hour, or at a dose ranging from 30 mg to 40 mg per day at a rate of 1 to 2 mg per hour, a dose ranging from 2.4 mg to 40 mg per day at a rate of 0.5 to 1 mg per hour, or a dose ranging from5 mg to 40 mg per day at a rate of 0.5 to 1 mg per hour, or at a dose ranging from 10 mg to 40 mg per day at a rate of 0.5 to 1 mg per hour, or at a dose ranging from 20 mg to 40 mg per day at a rate of 0.5 to 1 mg per hour, or at a dose ranging from 30 mg to 40 mg per day at a rate of. 0.5 to 1 mg per hour, a dose ranging from 2.4 mg to 30 mg per day at a rate of 0.1 to 2 mg per hour, or a dose ranging from 5 mg to 30 mg per day at a rate of 0.1 to 2 mg per hour, or at a dose ranging from 10 mg to 30 mg per day at a rate of .01 to 2 mg per hour, or at a dose ranging from 20 mg to 30 mg per day at a rate of .01 to 2 mg per hour, or a dose ranging from 2.4 mg to 30 mg per day at a rate of 1 to 2 mg per hour, or a dose ranging from 5 mg to 30 mg per day at a rate of 1 to 2 mg per hour, or at a dose ranging from 10 mg to 30mg per day at a rate of 1 to 2 mg per hour, or at a dose ranging from 20 mg to 30 mg per day at a rate of 1 to 2 mg per hour, a dose ranging from 2.4 mg to 30 mg per day at a rate of 0.5 to 1 mg per hour, or a dose ranging from 5 mg to 30 mg per day at a rate of 0.5 to 1 mg per hour, or at a dose ranging from 10 mg to 30 mg per day at a rate of 0.5 to 1 mg per hour, or at a dose ranging from 20 mg to 30 mg per day at a rate of 0.5 to 1 mg per hour, a dose ranging from 2.4 mg to 20 mg per day at a rate of 0.1 to 2 mg per hour, or a dose ranging from 5 mg to 20 mg per day at a rate of 0.1 to 2 mg per hour, or at a dose ranging from 10 mg to 20 mg per day at a rate of .01 to 2 mg per hour, or a dose ranging from 2.4 mg to 20 mg per day at a rate of 1 to 2 mg per hour, or a dose ranging from 5 mg to 20 mg per day at a rate of 1 to 2 mg per hour, or at a dose ranging from 10 mg to 20 mg per day at a rate of 1 to 2 mg per hour, a dose ranging from 2.4 mg to 20 mg per day at a rate of 0.5 to 1 mg per hour, or a dose ranging from5 mg to 20 mg per day at a rate of 0.5 to 1 mg per hour, or at a dose ranging from 10 mg to 20 mg per day at a rate of 0.5 to 1 mg per hour, a dose ranging from 5 mg to 10 mg per day at a rate of 0.1 to 2 mg per hour, a dose ranging from5 mg to 10 mg per day at a rate of 0.1 to 2 mg per hour, or a dose ranging from 2.4 mg to 10 mg per day at a rate of 1 to 2 mg per hour, or a dose ranging from 5 mg to 10 mg per day at a rate of 1 to 2 mg per hour, or a dose ranging from 2.4 mg to 10 mg per day at a rate of 0.5 to 1 mg per hour, a dose ranging from5 mg to 10 mg per day at a rate of 0.5 to 1 mg per hour, or a dose ranging from2.4 mg to 5 mg per day at a rate of 0.1 to 2 mg per hour, or a dose ranging from 2.4 mg to 5 mg per day at a rate of 0.5 to 1 mg per hour, a dose ranging from 2.4 mg to 5 mg per day at a rate of 1 to 2 mg per hour.

In any of the methods of preceding methods, compositions or uses, the dose of SFP administered achieves, increases or maintains Hgb levels in the dialysis patient at 9 g/dl or greater, or to at least about 10 g/dL, or to at least about 12 g/dL, or to at least about 14 g/dL. The methods of the invention increase or maintains the level of Hgb in an anemic subject so that the need for blood transfusions is reduced or eliminated.

Disclosed are of the preceding methods, compositions or uses wherein the SFP is in the form of a pyrophosphate citrate chelate, i.e. iron chelated or covalently bonded with citrate and pyrophosphate. Optionally, the SFP comprises iron in an amount of 7% to 11% by weight, citrate in an amount of about 14% to 30% by weight, and pyrophosphate in an amount of at least 10% by weight. In addition, the SFP may also comprise phosphate in an amount of 2% or less, or in an amount of 1% or less.

Furthermore, disclosed are any of the preceding methods, compositions or uses wherein the SFP is administered via dialysate and the dose of intravenously administered iron after SFP administration is at least 10% less than the dose of intravenously administered iron prior to SFP administration, or is at least 12.5% less than the dose of intravenously administered iron prior to SFP administration, or is at least 25% less than the dose of intravenously administered iron prior to SFP administration, or is at least 30% less than the dose of intravenously administered iron prior to SFP administration or is at least 33% less than the dose of intravenously administered iron prior to SFP administration, or is at least 50% less than the dose of intravenously administered iron prior to SFP administration, or is at least 75% less than the dose of intravenously administered iron prior to SFP administration, or is 100% less than the dose of intravenously administered iron prior to SFP administration.

Disclosed are any of the preceding methods, compositions or uses wherein the dose of intravenously administered iron after SFP administration ranges from at least about 10% less to about 50% less than the dose of intravenously administered iron prior to SFP administration, or ranges from at least about 10% less to about 25% less than the dose of intravenously administered iron prior to SFP administration, or ranges from at least about 25% less to about 50% less than the dose of intravenously administered iron prior to SFP administration, or ranges from at least about 50% less to about 75% less than the dose of intravenously administered iron prior to SFP administration, or ranges from at least about 75% less to about 100% less than the dose of intravenously administered iron prior to SFP administration, or ranges from about 10% less to about 100% less than the dose of intravenously administered iron prior to SFP administration, or from about 25% less to about 100% less than the dose of intravenously administered iron prior to SFP administration.

In any of the preceding methods, compositions or uses, the subject may be suffering from chronic kidney disease, optionally stage II, III, IV or V.

In addition, the disclosed are any of the preceding methods, compositions or uses wherein the subject is undergoing hemodialysis.

Disclosed are any of the preceding methods, compositions or uses wherein the subject is suffering from anemia of inflammation.

Disclosed are any of the preceding methods, compositions or uses wherein the subject is suffering from infection, optionally chronic infection.

Furthermore, disclosed are any of the preceding methods, compositions or uses wherein the subject is suffering from cancer, heart failure, autoimmune disease, sickle cell disease, thalassemia, blood loss, transfusion reaction, diabetes, vitamin B12 deficiency, collagen vascular disease, Shwachman syndrome, thrombocytopenic purpura, Celiac disease, endocrine deficiency state such as hypothyroidism or Addison's disease, autoimmune disease such as Crohn's Disease, systemic lupus erythematosis, rheumatoid arthritis or juvenile rheumatoid arthritis, ulcerative colitis immune disorders such as eosinophilic fasciitis, hypoimmunoglobulinemia, or thymoma/thymic carcinoma, graft vs. host disease, preleukemia, Nonhematologic syndrome (Down's, Dubowwitz, Seckel), Felty syndrome, hemolytic uremic syndrome, myelodysplasic syndrome, nocturnal paroxysmal hemoglobinuria, osteomyelofibrosis, pancytopenia, pure red-cell aplasia, Schoenlein-Henoch purpura, malaria, protein starvation, menorrhagia, systemic sclerosis, liver cirrhosis, hypometabolic states, congestive heart failure, chronic infections such as HIV/AIDS, tuberculosis, oseomyelitis, hepatitis B, hepatitis C, Epstein-bar virus or parvovirus, T cell leukemia virus, bacterial overgrowth syndrome, fungal or parasitic infections, and/or red cell membrane disorders such as hereditary spherocytosis, hereditary elliptocytosis, heriditray pyrpoikilocytosis, hereditary stomatocytosis, red cell enzyme defects, hypersplenism, immune hemolysis or paroxysmal nocturnal hemoglobinuria.

In addition, disclosed are any of the preceding methods, compositions or uses wherein the anemia is due to overt iron deficiency with depleted iron stores or a functional iron deficiency with adequate or excessive iron stores.

Disclosed are any of the preceding methods, compositions or uses wherein SFP is administered during hemodialysis within the hemodialysate solution. In addition, disclosed are any of the preceding methods, compositions or uses wherein SFP is administered during peritoneal dialysis within the peritoneal dialysis solution or wherein SFP is administered with parenteral nutrition within parenteral nutrition admixture. Disclosed are also any of the preceding methods, compositions or uses wherein the SFP administration is by oral, intravenous, intramuscular, subcutaneous, transbuccal, sublingual, intraperitoneal, intradermal or transdermal routes, or in conjunction with the dialysis solutions in patients with kidney disease undergoing hemodialysis or peritoneal dialysis.

Disclosed are any of the preceding methods wherein SFP is administered at a therapeutically effective dose that i) increases at least one marker of iron status selected from the group consisting of serum iron, transferrin saturation, reticulocyte hemoglobin, serum ferritin, reticulocyte count, and whole blood hemoglobin and ii) decreases the dose of ESA required to achieve or maintain target hemoglobin levels, or the need for transfusion of whole blood, packed red blood cell or blood substitutes. In addition, when any of the preceding methods, compositions or uses are carried out in a subject is suffering from non-anemic iron deficiency and administration of the therapeutically effective dose of SFP reduces fatigue, increases physical and cognitive ability, or improves exercise tolerance in the subject.

In addition, disclosed are any of the preceding methods, compositions or uses wherein SFP is administered in a therapeutically effective dose that will reduce or abolish the clinical manifestations of restless leg syndrome associated with iron deficiency.

Doses include, for example, about 1-50 mg SFP-iron per day at a rate of 0.1 to 2 mg per hour, given by any administration route, or about 2 to 48 mg per day, or 2 to 25 mg per day, or 2 to 10 mg per day, or 3 to 48 mg per day, or 3 to 25 mg per day, or 3 to 10 mg per day, or 4 to 48 or 4 to 25 mg per day, or 4 to 30 mg per day, or 4 to 10 mg per day, or 5 to 50 mg per day, 10 to 50 mg per day, 5 to 45 mg per day, 10 to 45 mg per day, 5 to 25 mg per day, 5 to 10 mg per day, 10 to 25 mg per day, 10 to 30 mg per day. Any of these doses may be administered at a rate of 0.1 to 2 mg per hour, or at a rate of 0.5 to 1 mg per hour, or at a rate ofl to 2 mg per hour.

### BRIEF DESCRIPTION OF DRAWING

**Figure 1** provides the percent change in prescribed ESA weekly dose equivalents for all subjects in the study described in Example 1. Triferic is the commercial name for the SFP composition administered to the study group.
**Figure 2** provides the mean IV dose of supplemental iron administered to all subjects in the study described in Example 1. Triferic is the commercial name for the SFP composition administered to the study group.
**Figure 3** provides the change in reticulocyte Hgb in all subjects in the study described in Example 1. Triferic is the commercial name for the SFP composition administered to the study group.
**Figure 4** provides the Hgb change compared from baseline in all subjects in the sturdy described in Example 1. Triferic is the commercial name for the SFP composition administered to the study group.
**Figure 5** provides the iron parameters during a single hemodialysis event in all subjects in the study described in Example 1. Triferic is the commercial name for the SFP composition administered to the study group. Pre HD is before the hemodialysis event and Post HD is after the hemodialysis event.
**Figure 6** provides the change in serum iron during hemodialysis in all subjects in the study described in Example 1. Triferic is the commercial name for the SFP composition administered to the study group. Pre HD is before the hemodialysis and Post HD is after the hemodialysis.
**Figure 7** provides the change in serum ferritin in all subjects in the study described in Example 1. Triferic is the commercial name for the SFP composition administered to the study group.

### DETAILED DESCRIPTION

Due to the adverse side effects associated with ESA therapy in dialysis patients, the lowest possible dose of ESA should be administered that will reduce or eliminate the need for blood transfusions. Furthermore, anemia of chronic kidney disease (CKD) is resistant to ESA treatment in 10-20% of patients (Babbitt and Lin, J Am Soc Nephrol 23: 1631-1634, 2012). The disclosure provide means of reducing ESA dose during treatment of dialysis patients while achieving or maintaining target Hgb levels in a patient while undergoing dialysis, by administering a therapeutically effective dose of SFP. The clinical study described in Examples 1 and 2 demonstrate that administration of SFP to dialysis patients that are hyporesponsive to ESA significantly reduced the ESA requirements in these dialysis patients. SFP administration reduced the need for ESA in the hyporesponsive patients without increasing iron stores.

Large doses of intravenously administered iron-carbohydrate complexes can also increase Hgb and reduce ESA need but lead to marked increase in tissue iron stores and inflammation as suggested by a marked increase in serum ferritin level (Besarab et al. J. Am, Soc. Nephrol. 11: 530-538, 2000). On the other hand, studies have shown that doses of SFP iron ranging from 100 to 120 µg per liter of hemodialysate maintain but do not increase serum ferritin levels (Gupta et al. J Am Soc Nephrol 2010; 21 (Renal Week 2010 Abstract Supplement): 429A 2010).

Therefore, the experimental evidence provided herein demonstrates a significant reduction in ESA requirements in dialysis patients that are hyporesponsive to ESA without an increase in tissue iron stores or inflammation. This highly unexpected result is likely due to the unique mode of action of SFP. It is now increasingly recognized that a major contributor to anemia in CKD patients is the anemia of inflammation that is due to disordered iron homeostasis largely mediated by the peptide hepcidin. Hepcidin excess likely accounts for impaired dietary iron absorption and impaired release of iron from reticuloendothelial iron stores. Produced by the liver and secreted into circulation, hepcidin binds and induces degradation of ferroportin, the iron exporter that is present on duodenal enterocytes, reticuloendothelial macrophages, and hepatocytes, thereby inhibiting iron entry into plasma. Inflammatory cytokines directly induce hepcidin transcription, presumably as a mechanism to sequester iron from invading pathogens, leading to the iron sequestration, hypoferremia, and anemia that are hallmarks of many chronic diseases including CKD. In CKD patients with hepcidin excess, large intravenous doses of iron would be predicted to have limited effectiveness because much of the iron is rapidly taken up by the liver and sequestered, and the remainder that is incorporated into red blood would be recycled ineffectively. In addition intravenous iron further increases hepcidin levels and worsens this phenomenon (Babitt & Lin, J. AM. Soc. Nephrol. 23: 1631, 1634, 2012). Administration of SFP appears to supply iron without worsening the anemia of inflammation. Without being bound by theory, SFP iron when delivered directly into the circulation by any parenteral route binds directly to the circulating iron carrier protein apotransferrin thereby forming monoferric or diferric transferrin, which then delivers iron directly to the red blood cell precursors in the bone marrow, bypassing processing by the reticulo-endothelial macrophages and hepatocytes.

It is expected that these findings apply to SFP delivered by any parenteral route. Parenteral routes for effective SFP delivery include but are not limited to intravenous, intramuscular, subcutaneous, intradermal, transdermal, transbuccal, sublingual, via hemodialysis when added to hemodialysis solutions, via peritoneal dialysis when added to peritoneal dialysis solutions, or in conjunction with parenteral nutrition admixtures when added to parenteral nutrition admixture.

### Erythropoiesis Stimulating Agents

Erythropoiesis is the generation of red blood cells (RBC) in the bone marrow. The term "erythropoiesis" is used herein to describe the process of proliferation and differentiation of hematopoietic stem cells (HSCs) and hematopoietic progenitor cells, leading to the production of mature red blood cells. "Erythropoiesis stimulating agents" (ESA) are agents that are capable of intimating and stimulating new red blood cell production.

Erythropoietin (EPO) is a circulating glycosylated protein hormone (34 KD) that is the primary regulator of RBC formation. Endogenous EPO is produced in amounts that correspond to the concentration of O₂ in the blood and is synthesized primarily in the kidney, although it is also made at lower levels in other tissues such as liver and brain.

The term "ESA" applies to all agents that augment erythropoiesis through direct or indirect action on the erythropoietin receptor. ESA includes endogenous human erythropoietin (GenBank Accession No. AAA52400; Lin et al. (1985) Proc. Natl. Acad. Sci. USA 82:7580-7584) and recombinant erythropoietin and erythropoietin-like substances such as EPOETIN products under the trade names of EPOGENTM (Amgen, Inc., Thousand Oaks, Calif.), EPREXTM (Janssen-Cilag. Ortho Biologics LLC) and NEORECORMONTM (Roche), ARANESP human recombinant darbepoietin (Amgen), PROCRITTM (Ortho Biotech Products, L.P., Raritan N.J.) and MIRCERA (Methoxy polyethylene glycol-epoetin beta; Roche), Peginesatide or Omontys® (Affymax Inc., Palo Alto, CA).

In some embodiments, ESA also includes water-soluble salts of transition metals such as manganese (Mn), cobalt (Co), and nickel (Ni); and also include titanium (Ti), vanadium (V), and chromium (Cr). Most, if not all, water-soluble salts of these transition metals are believed to stimulate red cell production when administered in suitable concentration in vivo. The salts thus include halides and most preferably chloride salts; carbonate and bicarbonate salts; sulfides, sulfites, and sulfates; nitrogen atom containing salts; oxides; and other conventional salt formats and formulations generally biocompatible and useful *in vivo.* In other embodiments, water-soluble salts of transition metals are excluded from the definition of ESA.

The daily doses administered to subjects in need may vary for the respective ESAs. It should be noted that doses for individual patients and patient groups will often differ from the daily defined dose and will necessarily have to be based on individual characteristics (*e.g*. age and weight) and pharmacokinetic considerations.

ESA are commonly used to treat anemia and are administered to subjects undergoing hemodialysis. Despite the positive effects ESA have on number of red blood cells and increasing Hgb levels, ESA treatment has many adverse effects and the FDA recommends administering the lowest dose of ESA sufficient to reduce or avoid the need for RBC transfusions.

### Hemoglobin

The methods disclosed herein administer a dose of SFP effective to increase or maintain hemoglobin (Hgb) levels in the subject. Hgb is a spheroidal protein that consists of four subunits, two pairs of identical polypeptide chains, each with a cleft or pocket on its exterior. The cleft contains a heme or iron-protoporphyrin group that is the site of oxygen uptake and release. A primary role of Hgb in RBCs is to carry O₂ to O₂-dependent tissues. Hgb found in RBCs is a tetrameric haem iron-containing protein. Anemic subjects have reduced Hgb levels and the methods of the invention comprise administering an effective dose of SFP to increase Hgb in subjects suffering from anemia. An effective dose of SFP increases Hgb levels and preferably the Hgb levels are increased to a level that is sufficient to adequately oxygenate the subject's tissues and/or reduce the need for blood transfusion. An exemplary sufficient Hgb in level for an anemic subject is about 10 g/dL. Normal Hgb levels for a human adult male range from 14 to 18 g/dL and for adult human women range from 12.0 to 16 g/dL. However, the target Hgb levels in a CKD patient receiving ESA is 9-11 g/dL.

Hemoglobin levels are determined as g/dL or g/L of whole blood of the subject. Hemoglobin levels may be measured using any method known in the art. For example, hemoglobin levels may be measuring using photometric detection of cyanmetahemoglobin, photmetic detection of azide metahemoglobin, or a peroxidase method (*e.g*. Crosby-Furth method) to name a few.

### SFP Compositions

Ferric pyrophosphate (Fe₄ O₂₁ P₆) has a molecular weight of 745.25. It has been used as a catalyst, in fireproofing synthetic fibers and in corrosion preventing pigments.

Food grade soluble ferric pyrophosphate (alternatively, "ferric pyrophosphate, soluble" or SFP) is an iron preparation of uncertain composition. No definite formula for its constitution is known. In general, it is described as "a mixture of ferric pyrophosphate and sodium citrate" or "a mixture of four salts (ferric and sodium pyrophosphates and ferric and sodium citrates)" or "ferric pyrophosphate that has been rendered soluble by sodium citrate." Soluble ferric pyrophosphate is known to have the properties described in Table 1.

**Table 1 - Properties of Conventional Ferric Pyrophosphate, Soluble**

| **Parameter** | **Observation** |
|---|---|
| Chemical Name | 1,2,3-Propanetricarboxylic acid, 2-hydroxy-, iron(3+) sodium salt (1:1:1), mixture with iron(3+) diphosphate |
| CAS Registry No. | 1332-96-3 |
| Appearance | Solid (may be plates, powder, or pearls, depending on the manufacturer |
| Color | Yellow-green to apple-green |
| Iron Content | 10.5% to 12.5% |
| Solubility in water | Exceeds 1 gram per mL |
| pH of a 5% solution | 5-7 |

TRIFERIC™ is a GMP grade SFP for pharmaceutical application in which ferric iron is covalently bonded to pyrophosphate and citrate. Triferic is more soluble and more stable compound than food grade SFP with a well characterized structure.

Disclosed are methods of administering SFP via parenteral delivery to anemic subjects. US Patent No. 6779468 describes parenteral administration of SFP, US Patent No. 6689275, describes compositions comprising SFP solutions, and US Patent No. 7,816,404 describes water-soluble SFP citrate chelate compositions.

Soluble ferric pyrophosphate may be obtained commercially. Food grade soluble ferric pyrophosphate (FCC-SFP) is an apple-green solid containing from about 10.5% to about 12.5% iron. According to the manufacturers, soluble ferric pyrophosphate is stable for as long as three years provided that it is protected against exposure to air and light.

Any of the methods disclosed herein may be carried out with water-soluble ferric pyrophosphate citrate chelate compositions, having, by weight, from about 7% to about 11% iron, from about 14% to about 30% citrate, from about 10% to about 20% pyrophosphate and about 2% or less phosphate. The chelate compositions may have, by weight, about 1.5% phosphate or less, or about 1% phosphate or less. In an aspect herein, the chelate compositions have, by weight, about 0.1% phosphate or less.

U.S Patent No. 7,816,404, provides methods of preparing SFP-citrate chelate compositions in accordance with GMP standards (GMP-SFP). Any of the methods disclosed herein may be carried out with water-soluble ferric pyrophosphate citrate chelate compositions, having, by weight, from about 7% to about 11% iron, at least 14% citrate, at least 10% pyrophosphate. The chelate compositions may have, by weight, about 1.5% phosphate or less, or about 1% phosphate or less. In an aspect of the invention, the chelate compositions have, by weight, about 0.1% phosphate or less.

### Dialysis and Solutions

The term "dialysis" includes both hemodialysis and peritoneal dialysis and is defined as the movement of solute and water through a semipermeable membrane (the dialyzer) which separates the patient's blood from a cleansing solution (the dialysate). It is a clinical treatment procedure by which metabolic by-products, toxins, and excess fluid are removed from the blood of a subject with chronic kidney disease (CKD) by transfer across a dialysis membrane. Dialysis may be conventionally performed as hemodialysis, in which a synthetic membrane constitutes the dialysis membrane, or as peritoneal dialysis, in which a patient's peritoneal membrane constitutes the dialysis membrane. The patient's plasma tends to equilibrate with the dialysate solution over time. The composition of the dialysate permits one to remove, balance or even infuse solutes from and into the patient. The electrochemical concentration gradient is the driving force that allows the passive diffusion and equilibration between the dialysate and the patient's blood compartment. Dialysis-related iron deficiency affects about 90% of CKD patients by six months of treatment. Disclosed is a method of administering SFP to patients suffering from CKD and undergoing dialysis via parenteral delivery including within hemodialysis solution or within peritoneal dialysis solution.

Hemodialysis refers to the use of a hemodialyzer to remove certain solutes from blood by virtue of their concentration gradients across a semipermeable membrane. The hemodialyzer, also referred to as an artificial kidney, is an apparatus by which hemodialysis is performed, blood being separated by the semipermeable membrane from a solution of such composition as to secure diffusion of certain solutes from the blood. The hemodialyzer can be used for ultrafiltration by which differences in fluid pressure bring about filtration of a protein-free fluid from the blood. Hemodialysis includes acute hemodialysis and maintenance hemodialysis.

Maintenance hemodialysis refers to long-term hemodialysis therapy for treatment of end stage renal failure. Patients on maintenance hemodialysis have been estimated to lose about 2 to 3 grams of iron per year, corresponding to approximately 6 ml per day (2 liters per year) blood loss from all sources (Eschbach et al. Ann. Intern Med. 87(6): 710-3, 1977).

During peritoneal dialysis, a patient's peritoneal membrane is used to exchange solutes and fluid with the blood compartment. Therefore, peritoneal dialysis is the treatment of uremia by the application of kinetic transport of water-soluble metabolites by the force of diffusion and the transport of water by the force of osmosis across the peritoneum. The peritoneum is the largest serous membrane of the body (approximately 2 m² in an adult). It lines the inside of the abdominal wall (parietal peritoneum) and the viscera (visceral peritoneum). The space between the parietal and visceral portions of the membrane is called the "peritoneal cavity." Aqueous solutions infused into the cavity (dialysate) contact the blood vascular space through the capillary network in the peritoneal membrane. The solution infused into the peritoneal cavity tends to equilibrate with plasma water over time and it is removed at the end of one exchange after partial or complete equilibration. The composition of the dialysate allows for the removal, balance or infusion of solutes from and into the patient. The electrochemical concentration gradient is the driving force that allows the passive diffusion and equilibration between the dialysate and blood compartment.

In general, the pharmaceutical compositions disclosed herein can be prepared by conventional techniques, as are described in Remington's Pharmaceutical Sciences, a standard reference in this field (Gennaro A R, Ed. Remington: The Science and Practice of Pharmacy. 20th Ed. Baltimore: Lippincott, Williams & Williams, 2000). For therapeutic purposes, the active components of this invention are ordinarily combined with one or more excipients appropriate to the indicated route of administration. A "dialysate solution or dialysate" is the solution used, on the opposite side of the membrane from the patient's blood, during dialysis. Dialysate is conventionally provided for use in either peritoneal dialysis (in which the peritoneal membrane constitutes the dialysis membrane) or hemodialysis (in which a synthetic membrane constitutes the dialysis membrane). Hemodialysate is generally prepared from two dry powder concentrates, including acid ("A") and base ("B") concentrates, which are reconstituted in treated water before use, or from two aqueous concentrates. The A concentrate, containing an organic acid and electrolytes and osmotic agents other than bicarbonate, is mixed with B concentrate containing bicarbonate and treated water in a dialysis machine to make the final hemodialysate. Peritoneal dialysate is a premixed solution of osmotic agents, electrolytes, and water that is used in dialysis without further constitution.

Presently, hemodialysis machines utilize an automated proportioning system to mix salts in deionized water in specific proportions to generate the final dialysate solution. The dialysate concentrates are usually supplied by the manufacturer either as a solution ready to use or as a premixed powder that is added to purified water in large reservoirs. The concentrates are pumped into a chamber in the dialysis machine where they are mixed with purified water to make the final dialysate solution.

Generally, the ionic composition of the final dialysate solution for hemodialysis is as follows: Na⁺ 132-145 mmol/L, K⁺ 0-4.0 mmol/L, Cl⁻ 99-112 mmol/L, Ca⁺⁺ 2.0-3.5 mEq/L, Mg⁺² 0.25-0.75 mmol/L, Dextrose 100-200 mg/dL. The correction of metabolic acidosis is one of the fundamental goals of dialysis. In dialysis, the process of H⁺ removal from the blood is mainly achieved by the flux of alkaline equivalents from the dialysate into the blood, thereby replacing physiological buffers normally utilized in the chemical process of buffering. In dialysis practice, base transfer across the dialysis membrane is achieved by using acetate or bicarbonate containing dialysate. In "Bicarbonate dialysis" the dialysate contains 27-35 mmol/L of bicarbonate and 2.5-10 mmol/L of acetate. On the other hand, in "Acetate dialysis" the dialysate is devoid of bicarbonate and contains 31-45 mmol/L of acetate. Ferric pyrophosphate is compatible with both acetate and bicarbonate based hemodialysis solutions.

The peritoneal dialysis fluid usually contains Na⁺ 132-135 mmol/L, K⁺ 0-3 mmol/L, Ca⁺⁺ 1.25-1.75 mmol/L, Mg⁺⁺ 0.25-0.75 mmol/L, Cl⁻ 95-107.5 mmol/L, acetate 35 mmol/L or lactate 35-40 mmol/L and glucose 1.5-4.25 gm/dL.

### Patient populations

The method disclosed herein may be carried out on dialysis patients that are hypo-responsive to ESA therapy. The term "hyporesponsive to ESA" refers to patients that require a significant increase in ESA dosing to maintain target Hgb levels. These patients may or may not be suffering from anemia. Hyporesponsiveness to erythropoietin or ESA-resistant anemia refers to the presence of at least one of the following conditions: i) a significant decrease in Hgb levels at a constant dose of ESA treatment, ii) a significant increase in the ESA dose requirement to achieve or maintain a certain Hgb level, iii) a failure to raise the Hgb level to the target range despite the ESA dose equivalent to erythropoietin greater than 150 IU/kg/week or 0.75 mg/kg/week of darbepoeitn-alpha or continued need for such high dose of ESA to maintain the target Hgb level. For example, approximately 5-10% of patients with CDK demonstrate hyporesponsiveness to ESA, defined as a continued need for greater than 300 IU/kg per week erythropoietin or 1.5 mµg/kg per week darbepoetin administered by the subcutaneous route (Johnston et al., Nephrology. 2007 Aug;12(4):321-30).

In the clinical study described in Examples 1 and 2, about 20% of study patients, the relatively ESA hyporeponsive group, was receiving greater than 13,000 Unit of ESA per week at the start of the study (Note: Patients with ESA doses exceeding 45,000 units per week were excluded from the study). The method disclosed herein may be particularly useful in maintaining and/or increasing Hgb levels in dialysis patients that are hyporesponsive to ESA and in subjects suffering from ESA-resistant anemia.

The term "anemia" refers to a condition when the number of red blood cells and/or the amount of Hgb found in the red blood cells is below normal, and may be acute or chronic. For example, the term "anemia" includes but is not limited to iron deficiency anemia, renal anemia, anemia of chronic diseases/inflammation, pernicious anemia such as macrocytic achylic anemia, juvenile pernicious anemia and congenital pernicious anemia, cancer-related anemia, chemotherapy-related anemia, radiotherapy-related anemia, pure red cell aplasia, refractory anemia with excess of blasts, aplastic anemia, X-lined siderobalstic anemia, hemolytic anemia, sickle cell anemia, anemia caused by impaired production of ESA, myelodysplastic syndromes, hypochromic anemia, microcytic anemia, sideroblastic anemia, autoimmune hemolytic anemia, Cooley's anemia, Mediterranean anemia, Diamond Blackfan anemia, Fanconi's anemia and drug-induced immune hemolytic anemia. Anemia may cause serious symptoms, including hypoxia, chronic fatigue, lack of concentration, pale skin, low blood pressure, dizziness and heart failure.

Normal Hgb ranges for humans are about 14-18 g/dl for men and 12-16 for women g/dl with the average Hgb value for men at about 16 g/dL and for women at about 14 g/dL. Anemia may be considered a drop of Hgb levels below about 12 g/dL and severe anemia may be considered a drop in Hgb below about 8 g/dL. The grading system for anemia is provided in Table 3 below.

**Table 3 Grading System for Anemia* (Groopman & Itri, J. Natl. Canc. Inst. 91(19): 1616-1634. 1999)**

| **Severity** | **WHO** | **NCI** |
|---|---|---|
| Grade 0 (WNL)# | ≥ 11.0 g/dL | WNL |
| Grade 1 (mild) | 9.5-10.9 g/dL | 10.0 g/dL to WNL |
| Grade 2 (moderate) | 8.0-9.4 g/dL | 8.0- 10 g/dL |
| Grade 3 (serious/severe) | 6.5-7.9 g/dL | 6.5-7.9 g/dL |
| Grade 4 (life threatening) | <6.5g/dL | <6.5g/dL |

| | | |
|---|---|---|
| * WHO = World Health Organization; NCI = National Cancer Institute WNL = within normal limits. # WNL hemoglobin values are 12.0-16.0 g/dL for women and 14.0-18.0 g/dL for men. | | |

Anemia may be assessed by assays well-known in the art such as a Complete Blood Count (CBC) test that measures the red blood cell (RBC) count, hematocrit, Hgb levels, white blood cell count (WBC), differential blood count, and platelet count. The first three parameters, the number of RBCs, hematocrit, and hemoglobin levels are the most commonly used in determining whether or not the patient is suffering from anemia.

Total iron binding capacity (TIBC) measures the level for transferrin in the blood. Transferrin is a protein that carries iron in the blood and a higher than normal TIBC value is a sign of iron-deficiency anemia and a lower than normal level indicates anemia of inflammation, pernicious anemia, or hemolytic anemia. Additionally, tests for anemia include reticulocyte count, serum ferritin, serum iron, transferrin saturation, reticulocyte Hgb, percentage of hypochromic RBCs, soluble transferrin receptor, direct or indirect Coombs' test, indirect bilirubin levels, serum haptoglobin, vitamin B12 levels, folate levels, and urine Hgb. The upper normal limit of reticulocytes (immature red blood cells) is about 1.5%, a low count suggests problems with the bone marrow and a high count suggests hemolytic anemia (e.g., the patient's body is attempting to make up for a loss of RBCs).

The anemic subject may have undergone or is currently undergoing cancer therapies (e.g. chemotherapy and radiation), bone marrow transplant hematopoietic stem cell transplant, exposure to toxins, hemodialysis, peritoneal dialysis, treatment with parenteral nutrition, gastrectomy surgery and/or pregnancy.

Anemia of inflammation is a type of anemia that commonly occurs with chronic, or long-term, illnesses or infections. The cytokines produced due to inflammation interfere with the body's ability to absorb and use iron and anemia results. In addition, cytokines may also interfere with the production and normal activity of erythropoietin. Hepcidin is thought to play a role as a key mediator of anemia of inflammation. The subject of any of the methods of the invention may be suffering from anemia of inflammation secondary to underlying disease state that is one of the following: chronic kidney disease, cancer, infectious diseases such as tuberculosis, HIV, endocarditis (infection in the heart), osteomyelitis (bone infection), hepatitis, inflammatory diseases such as rheumatoid arthritis and, lupus erthematosis, diabetes, heart failure, degenerative joint disease, and inflammatory bowel disease (IBD). IBD, including Crohn's disease, can also cause iron deficiency due to poor absorption of iron by the diseased intestine and bleeding from the gastrointestinal tract.

### Increased Ferritin Levels

CKD patients have increased iron losses due to chronic bleeding, frequent phlebotomy and blood trapping in the dialysis apparatus; and these patients have impaired dietary iron absorption Therefore, intravenous iron is commonly administered to hemodialysis patients because of the impaired dietary absorption (Babitt & Lin, J. Am. Soc. Nephrol. 23: 1631, 1634, 2012). CKD patients also have functional iron deficiency characterized by impaired iron release from body stores that is unable to meet the demand for erythropoiesis (reticulo-endothelial blockade). Thus, CKD patients may have normal or high serum ferritin levels and the treatment with IV iron is likely to have poor effectiveness and has the potential for adverse effects including liver iron overload (Vaziri, Am J. Med. 125(10);951-2, 2012), oxidant-mediated tissue injury due to excess iron deposition and increased risk of infection. Furthermore, CKD patients have an increase in hepcidin, the main hormone responsible for maintaining systemic iron homeostatis. It is predicted that in patients with excess hepcidin, administration of IV iron would have limited effectiveness because much of the iron is taken up by the liver and sequestered (Rostoker, Am J. Med. 125(10):991-999, 2012), and the remainder would be taken up by the red blood cells and would be recycled ineffectively. Plus, IV iron administration would further increase hepcidin levels. (Babitt & Lin, J. AM. Soc. Nephrol. 23: 1631, 1634, 2012)

While the methods disclosed herein provide for treating dialysis patients with hyporesponseness to ESA, these methods also reduce the dose of intravenously administered iron in these ESA hyporesponsive patients. SFP when delivered directly into the circulation by any parenteral route binds directly to circulating iron carrier protein apotransferrin thereby forming monoferric or diferric transferrin, which then delivers iron directly to the red blood cell precursors in the bone marrow, bypassing processing by the reticuloendothelial macrophages and hepatocytes. Therefore, SFP increases or maintains the Hgb levels in ESA hyporesponsive dialysis patients without increasing the iron stores (ferritin levels) in the patient and is an effective treatment or prevention of anemia of inflammation, a major contributor to the anemia seen in CKD patients.

With intravenous iron therapy, serum iron, transferrin and ferritin levels have to be regularly monitored to estimate the need for iron and to measure a response to the therapy. Iron excess in dialysis patients generally refers to serum ferritin level of more than 500 µg/liter or more than 800 µg/liter or more than 1000 µg/liter. Excess ferritin levels may result in or exacerbate ESA resistance. Finally, there is also a concern about potential iron overload with intravenous therapy, since the risk of infection and possibly cancer are increased in patients with iron overload (Weinberg, Physiol. Rev. 64(1): 62-102, 1984).

As demonstrated in the clinical study provided in Example 1, administration of SFP increases or maintains Hgb level in subjects suffering from anemia while not increasing serum ferritin levels. Thus, the methods of the invention are useful for reducing the dose of ESA administered to dialysis patient that are hyporesponsive to ESA, SFP also reduces the dose of intravenous iron administered to dialysis patients as described in Example 2.

### EXAMPLES

### Example 1

### Physiological Iron Maintenance in ESRD Subjects by Delivery of Soluble Ferric Pyrophosphate (SFP) via Hemodialysate: The PRIME Study

### OBJECTIVES

This study was designed to investigate the hypothesis that regular administration of soluble ferric pyrophosphate (SFP) administered via hemodialysate safely and effectively prevents the development of iron deficiency in end-stage renal disease (ESRD) subjects receiving maintenance hemodialysis (HD). Subjects randomized to receive dialysis using standard dialysate (placebo) were compared with subjects dialyzed using dialysis solution containing SFP.

The primary endpoint of the study was to determine the efficacy of SFP in maintaining iron sufficiency and thereby reducing the dose of erythropoiesis stimulating agents (ESAs) required to maintain hemoglobin (Hgb) levels. In addition, the primary endpoint was to determine the safety of SFP delivered via dialysate by assessing adverse events (AEs), physical examinations and vital signs, and laboratory tests (red and white cell characteristics, blood chemistries).

The secondary endpoint of the study was to compare the two study groups (standard versus SFP dialysate) for the following parameters, i) iron delivery, ii) the distribution of changes from baselinein prescribed ESA dose and iii) the amount of supplemental intravenous (IV) iron needed, iv) stability of Hgb over time (maintenance of Hgb between 95-115 g/L), and v) variability of Hgb. Iron delivery to the erythron was estimated by Hgb generation in response to erythropoietin (ESA response index [ERI], calculated as ESA dose/Hgb). The ERI was also to be divided by body weight in kilograms to obtain a modified ERI (ERI/kg).

### METHODS

**Number of Subjects:** CKD-HD subjects were randomized in a 1:1 ratio to receive hemodialysis using either dialysate containing SFP-iron (Fe-HD) or control iron-free dialysate (C-HD) (placebo) in a double blind fashion at every dialysis session. The total duration of the study was 36 weeks plus a one-week follow-up after the last study drug treatment. In the treatment phase, Hgb was measured weekly and serum ferritin and transferrin saturation (TSAT) determined every other week. These values were used to determine the ESA and IV iron prescriptions. An independent, blinded, Central Anemia Management Center (CAMC) facilitated consistent adherence to protocol-specified anemia management in regard to the appropriate application of the ESA dose adjustment algorithm for all study subjects across all study sites. Safety parameters were carefully monitored throughout the trial.
Planned: 100
Actual: 108 (Randomized: 108; Received study drug: 104).

**Table 4. Study Design: Iron Management and Monitoring of Iron Status**

| SCREENING/ PRE-TREATMENT | TREATMENT | | FOLLOW-UP |
|---|---|---|---|
| | 1st to 4th weeks | 5th to 36th weeks | Week 37 |
| 1. It is preferred to enroll subject just after their usual monthly iron studies | C-HD group: | C-HD group: | Iron dialysate and IV iron discontinued |
| | Control iron-free dialysate | Control iron-free dialysate | |
| | | Fe-HD group: | |
| 2. All oral iron and IV iron discontinued at least two weeks prior to randomization; and no ESA changes are allowed within four weeks prior to randomization | Fe-HD group: | Iron (SFP)-containing dialysate | |
| | Iron (SFP)-containing dialysate | Subjects in any group: | At end of Week 37, all subjects may be switched to IV iron and/or oral iron, as per local study site |
| | | A. ESA changes & IV iron per protocol | |
| 3. Randomize subject to control iron-free dialysate (C-HD) or iron (SFP)-containing dialysate (Fe-HD) | No IV iron or ESA change during first four weeks after randomizationa | B. Conversion to standard bicarbonate concentrate if iron overload develops or bacteremia/fungemia | |

| | | | |
|---|---|---|---|
| ESA dose reduction permitted every 2 weeks for rising Hgb or persistently high Hgb, or ESA may be withheld immediately if Hgb > 120 g/L. | | | |

**Diagnosis and Main Criteria for Inclusion:** For the study, Male and female subjects ≥ 18 years of age with ESRD on maintenance hemodialysis (HD) 3-4 times per week via an arteriovenous (AV) fistula or graft, with mean Hgb in the range of 95-120 g/L, mean ferritin 200-1000 µg/L, mean TSAT 15-40%, and prescribed ESA 4,000-45,000 U/week epoetin or 12.5-200 g/week darbepoetin (stable ESA dosing). In addition, the subjects had a history of IV iron during 6 months preceding randomization, and adequate dialysis and dialyzer blood flow.

The main exclusion criteria were ongoing infectious process, > 600 mg IV iron during the 6 weeks prior to randomization, changes in ESA dosage prior to randomization, active bleeding from any site other than AV fistula or graft, scheduled surgery or kidney transplant, RBC or whole blood transfusion within 12 weeks prior to randomization, active inflammatory disorder and subjects who were anticipated to be unable to complete the entire study.

**Table 5 Demographics for the Entire Study**

| | **SPF (N = 52)** | **Placebo (N = 51)** |
|---|---|---|
| Age year (SD) | 59.3 (12.6) | 58.7 (13.7) |
| Male n (%) | 29 (55.6) | 34 (66.7) |
| Female n (%) | 23 (44.2) | 17 (33.3) |
| Race: | | |
| White n (%) | 31 (59.6) | 32 (62.7) |
| Other n (5) | 21 (40.4) | 19 (37.3) |
| Post HD weight (mean (SD) kg | 86.0 (17.2) | 82.8 (17.6) |
| Time since first HD (months) | 47.6 (49.2) | 44.9 (63.7) |
| Prescribed ESA - Epoetin U/week (SD) | 9588 (5681) | 8998 (5401) |
| Prescribed ESA - Darbepoiten ug/week | | 18.8 |
| IV iron in previous 6 weeks (mg) | 102.1 (128.6) | 96.4 (111.7) |

**Test Product, Dose and Mode of Administration:** 2 µM (110 µg/L) SFP-iron in final dialysate was delivered to the test subjects through HD delivery system. Study Drug was provided as premixed jugs of SFP in bicarbonate concentrate, and is commercially known as TRIFERIC™.

**Reference Therapy, Dose and Mode of Administration:** Placebo or standard solution lacking iron was delivered to the control subjects. The placebo was provided as liquid bicarbonate concentrate.

**Method of Assigning Subjects to Treatment Groups:** A stratified, blocked randomization schema was used to assign subjects to treatment. Randomization was stratified by baseline ESA dose (the weekly dose as of the time of randomization), with subjects receiving ≤ 13,000 units/week epoetin (or ≤40 µg/week darbepoetin) (designated Stratum I); separately from subjects receiving >13,000 units/week epoetin (or >40 µg/week darbepoetin) (designated Stratum II). Stratum II patients were considered hyporesponsive to ESA. Within each stratum, subjects were randomized in a 1:1 ratio to SFP or placebo using an appropriate block size. At the time of randomization, subjects were assigned unique subject identification numbers. Numbers were to be assigned in consecutive increasing order without replacement or reuse of any assigned number.

**Table 6. IV Iron Administration Algorithm to Determine IV Iron Administration Based on Most Recent Serum Ferritin and Pre-Dialysis TSAT (with Confirmation)**

| **Ferritin (µg/L^{)a}** | **Pre-Dialysis TSAT^{b}** | **"Administration" of IV Iron^{*a*,*b*}** |
|---|---|---|
| Iron Sucrose Injection (e.g., Venofer®) 100 mg in 5 mL Unit Dose | | |
| <100 | <15% | 100 mg (5 mL), 100 mg (5 mL) & 100 mg (5 mL) at 3 consecutive dialysis sessions = total dose 300 mg |
| | 15-<25% | 100 mg (5 mL) & 100 mg (5 mL) at 2 consecutive dialysis sessions = total dose 200 mg |
| | 25-<50% | 100 mg (5 mL) at a single dialysis session = total dose 100 mg |
| | ≥50% | 0 |
| 100-200 | <15% | 100 mg (5 mL) & 100 mg (5 mL) at 2 consecutive dialysis sessions = total dose 200 mg |
| | 15-<25% | 100 mg (5 mL) at a single dialysis session = total dose 100 mg |
| | ≥25% | 0 |
| >200 | <15% | 100 mg (5 mL) at a single dialysis session = total dose 100 mg |
| | ≥15% | 0 |

For the data in Table 6, prior to IV iron dosing based on this algorithm, the ferritin and TSAT was confirmed by consecutive repeat values any time ≥ 1 day and ≤ 2 weeks after the first value (*a* in Table 6). In addition, A new "administration" of iron could be started sooner than four weeks after the start of the last "administration," or given as frequently as once every week, provided that Hgb has decreased by ≥ 15 g/L from the subject's baseline Hgb, or serum ferritin has remained <100 µg/L for ≥ 8 weeks, or the ESA dose according to the ESA Dose had increased by ≥50% compared to the subject's baseline ESA dose (*b* in Table 2).

**ESA Dose Adjustment:** Changes in type of prescribed ESA (e.g., epoetin vs. darbepoetin) and route of administration were prohibited during Screening (Weeks -2 and -1), and for a total of 6 weeks prior to anticipated randomization. Changes in the prescribed ESA dose within 4 weeks prior to randomization were prohibited, as was a prescribed ESA dose at the time of randomization that was >25% higher or lower than the prescribed dose at 6 weeks prior to randomization.

ESA dosing titrations for renal anemia during the course of the study were consistent with Food and Drug Administration (FDA) and the Centers for Medicare & Medicaid Services (CMS) guidelines. In the interest of subject safety and in recognition of FDA warnings and guidance issued November 8, 2007 and FDA guidance issued June 24, 2011, the ESA dose was interrupted or reduced when the Hgb level reached or exceeded 110 g/L and ESA was discontinued when the Hgb level reached or exceeded 120 g/L. During Week 1 through Week 4 of the Treatment Period, changes in the ESA dose could be made based on these criteria; but otherwise changes in ESA dose, type of ESA, and route of administration were prohibited.

Beginning at Week 5, the ESA dose could be adjusted. The ESA dose was adjusted based on the average of the subject's three Hgb values over a two consecutive week period, taking into consideration the rate of change in Hgb, according to Table 7. All decisions of change in ESA dose were made under the direction of the independent CAMC working with each study site's anemia manager, along with investigator discussions with the Sponsor medical monitor as needed.

**Table 7. ESA Dose Adjustment Algorithm (CAMC)**

| **Absolute Hgb (g/L) Based on Mean of Two Weekly Values** | **Mean Weekly Change in Hgb (g/L) over Prior Two Weeks (Most Recent Hgb minus Hgb Two Weeks Prior)/2** | | |
|---|---|---|---|
| | **Hgb Decrease > 5 g/L per Week** | **Hgb Change ≤ 5 g/L per Week** | **Hgb Increase > 5 g/L per Week** |
| ≤ 90 | 67% ESA ↑ | 33% ESA ↑ | ESA ↔ |
| > 90 to ≤ 100 | 50% ESA ↑ | 25% ESA ↑ | 12.5% ESA ↓ |
| >100 to ≤ 105 | 33% ESA ↑ | 12.5% ESA ↑ | 25% ESA ↓ |
| > 105 to ≤ 110 | 25% ESA ↑ | ESA ↔ | 33% ESA ↓ |
| > 110 to ≤ 115 | ESA ↔ | 12.5% ESA ↓ | 33% ESA ↓ |
| >115 to ≤ 120 | 12.5% ESA ↓ | 25% ESA ↓ | 50% ESA ↓ |
| > 120 | Withhold dosing until Hgb < 120 g/L, checking Hgb weekly, and then resume at a 25% ESA dose reduction | | |

| | | | |
|---|---|---|---|
| Note 1: No ESA dose should have been be changed until confirmed by the study's CAMC. Note 2: Actual prescribed dose of ESA per this algorithm could be rounded up or down (whichever is closer) by a maximum of 10% from the calculated dose if needed to minimize waste from the ESA product packaging. | | | |

During Week 5 through Week 36 of the Treatment Period, the ESA dose could be decreased every 2 weeks for rising Hgb or persistently high Hgb, with the exception of the Hgb rising above 120 g/L. In such cases, the ESA dose could be held immediately regardless of when the most recent dose change occurred. The ESA dose could only be increased every 4 weeks, with two exceptions. If ESA therapy was withheld due to a Hgb value > 120 g/L, it could be resumed as soon as the Hgb value was confirmed to have decreased to <120 g/L. Also, the ESA dose could be increased in 2 weeks if a Hgb decrease exceeded 7.5 g/L per week over the prior two weeks, or if the subject was considered at risk for requiring a blood transfusion.

The ESA dose could be adjusted according to the local unit conventions during follow-up (Week 37 or one week after last study drug exposure in case of Early Termination).

### CRITERIA FOR EVALUATION:

**Efficacy:** The primary efficacy endpoint was the percent change from baseline in the prescribed ESA dose required to maintain Hgb in the target range, adjusted for Hgb. The secondary efficacy endpoints were as follows: i) The amount of supplemental IV iron needed. ii) Iron delivery to the erythron as estimated by Hgb generation in response to erythropoietin (ESA response index, or ERI, calculated as prescribed ESA dose/Hgb). The ERI was also divided by body weight in kilograms to obtain a modified ERI (ERI/kg). iii) Maintenance of Hgb in the range of 95 to 115 g/L. iv) Variability of Hgb. v) The distribution of changes from baseline in ESA dose at end-of-treatment (≥ 25%, 10 to < 25%, > -10 to < 10%, > -25 to -10%, and ≤ -25%). **Safety:** Safety and tolerability of the drug were determined by the incidence and severity of adverse effects (AE), clinical laboratory measures, and clinically significant changes in physical examinations and vital signs.

### STATISTICAL METHODS:

**Efficacy:** Statistical analyses of the primary efficacy endpoint were conducted for both the modified intent-to-treat (MITT) and efficacy-evaluable populations. The primary analysis was performed using an analysis of covariance (ANCOVA) model with percent change from baseline in ESA as the response variable, treatment as the factor, and baseline Hgb as a covariate, with statistical significance assessed by the treatment p-value. The p-value for the treatment difference and a test of the treatment effect were performed at the two-tailed 5% significance level.

Secondary endpoints were analyzed using the MITT population. Continuous variables such as ERI, ERI/kg, and amount of supplemental IV iron were summarized using descriptive statistics, and analyzed using Wilcoxon rank-sum tests. The change from baseline in the prescribed ESA dose at end-of-treatment was categorized for each subject as being ≥25%, 10 to <25%, > -10 to < 10%, > -25 to -10%, and ≤ -25% and analyzed using the Cochran Mantel Haenzel (CMH) test stratified by baseline ESA dose (>13,000 or ≤13,000 U/week). The subjects who maintained Hgb levels (between 95-115 g/L, inclusive) were summarized using descriptive statistics by time point. The CMH chi-square test controlling for baseline ESA dose was used to compare the proportion of subjects maintaining Hgb levels between treatment groups. For each subject, the baseline and weekly post-baseline Hgb results over time were fit to a linear regression model, and this model was used to define the subject's temporal trend in Hgb (slope of regression line) and the Hgb variability as assessed by the residual standard deviation. Hgb variability was also assessed using the average absolute Hgb change.

The analyses of primary and selected secondary endpoints were done for the MITT and efficacy-evaluable populations by baseline ESA dose stratum as exploratory analyses.

**Safety Measures:** The number and percentage of subjects with adverse effected (AEs) were summarized. At each level of subject summarization, subjects who reported one or more AEs within a system organ class (SOC) and/or preferred term level were only counted once for that level. Tabular summaries included all treatment-emergent AEs, treatment-emergent AEs by severity, and treatment-emergent AEs by relationship to treatment, treatment-emergent serious adverse events (SAEs), treatment-emergent AEs leading to temporary withholding of study drug, and treatment-emergent AEs leading to permanent study drug discontinuation. All physical examination results were presented in a data listing. Any deaths occurring on study were summarized and presented in a listing.

The intra-dialytic hypotension (IDH) episodes based on a decrease in systolic blood pressure (SBP) meeting the protocol definition of IDH and IDH episodes based on premature termination or interruption of dialysis due to hypotension were summarized by treatment group for each four-week interval from Weeks 1-4 to Weeks 33-36. The number of events and the number and percentage of subjects with each symptom of IDH, and the number of events and the number and percentage of subjects that required each intervention for IDH, were summarized. The number and percentage of subjects with hypersensitivity reactions to IV iron or SFP were summarized by treatment group.

Clinical laboratory parameters and vital signs were summarized for each applicable study visit using descriptive statistics.

### SUMMARY OF RESULTS

**Subject Disposition:** Of the 103 subjects (52 in the SFP group, 51 in the placebo group) who received study drug, the majority of both groups completed the study (SFP, 41 subjects, 78.8%; and placebo, 40 subjects, 78.4%).

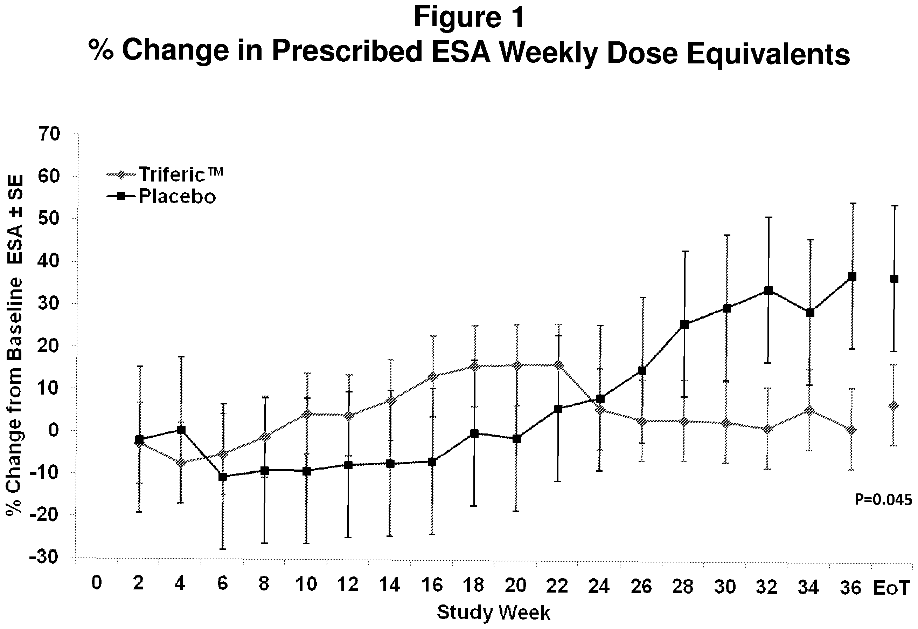

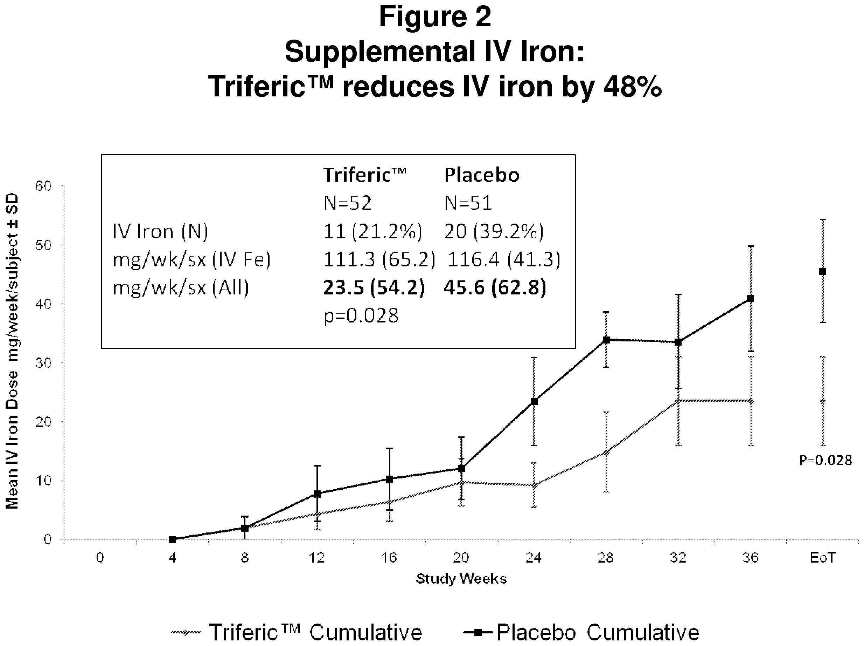

**Efficacy Results:** The percent change in the prescribed ESA dose required to maintain Hgb in the target range from baseline to end-of-treatment, the primary efficacy analysis, was statistically significantly smaller in the SFP group compared to the placebo group (4.9% vs. 39.8%, p=0.045) in the MITT population (see Fig. 1). As shown in Fig. 2, the mean dose of supplemental IV iron at end-of-treatment (a secondary efficacy analysis), was statistically significantly lower (p=0.028) in the SFP group (23.5 mg/week) than in the placebo group (45.6 mg/week). At end-of-treatment, in the MITT population, fewer subjects received supplemental IV iron in the SFP group (11 subjects, 21.1%) than in the placebo group (20 subjects, 39.7%). The mean change in the ERI/kg and the mean change in the ERI from baseline to end-of-treatment (both secondary efficacy analyses) were both smaller in the SFP group than in the placebo group in the MITT population; however, in neither case was the difference statistically significant.

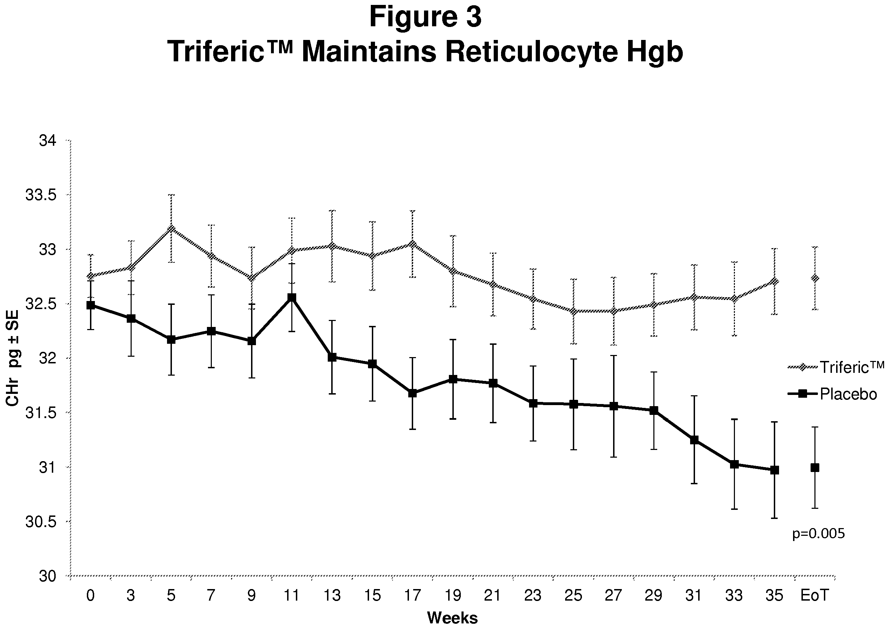

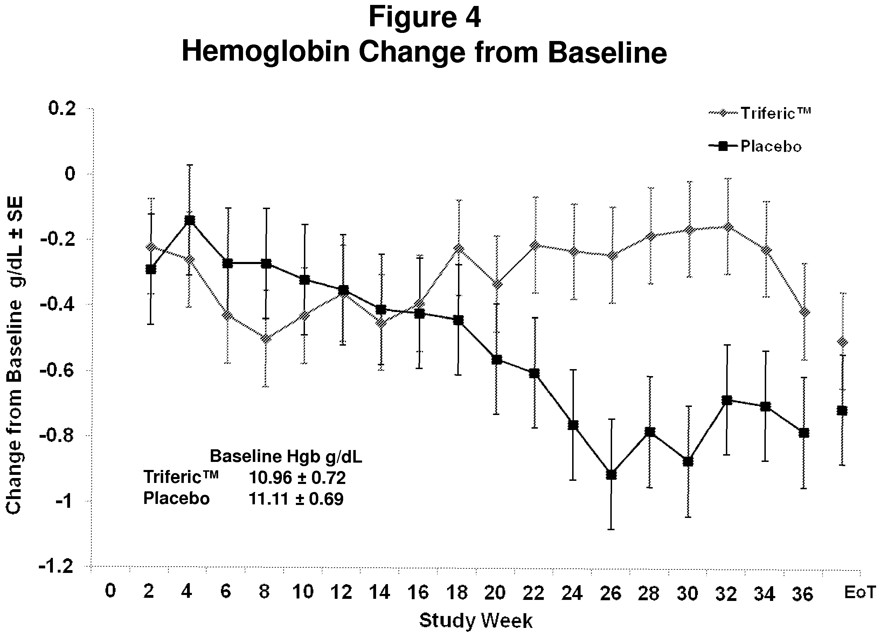

The distribution of changes from baseline in the prescribed ESA dose at end-of-treatment for the MITT population demonstrated that the percentages of subjects with a 25% increase in the prescribed ESA dose (30.8% and 39.7%, respectively, in the SFP and placebo groups) were similar to the percentages of subjects with a 25% decrease (30.8% and 29.4%, respectively, in the SFP and placebo groups). As shown in Fig. 3, reticulocyte hemoglobin values were higher in the SFP group than placebo at end-of-treatment. Hgb concentration was stable during the study, with both groups in the MITT population showing similar variability. Hgb levels were maintained within the 95 to 115 g/L range for >80% of time on study for similar percentages of subjects in the SFP (57.7%) and placebo (58.8%) groups in the MITT population (see Fig. 4).

**Table 8: Primary Endpoint: SPF Spares ESA Use Compared to Placebo**

| | SFP N=52 | | Placebo (N = 51) | |
|---|---|---|---|---|
| Baseline Prescribed ESA | U/wk (SD) | % Change from Baseline | U/wk (SD) | % Change from Baseline |
| Mean (SD) | 9483 (5414) | | 9206 (5500) | |
| End of Treatment ESA Dose (U/wk) | | | | |
| Mean (SD) | 9871 (7523) | 7.3 (67.66) | 12,628 (13,967) | 37.3 (106.9) |
| LS mean (SE) % Change from Baseline | 4.9 (12.1) | | 39.8 (12.2) | |
| 95% Cl LS mean | -19.1, 28.8 | | 15.7, 64.0 | |
| LS mean difference from Placebo | -35.0 (17.20) | | | |
| 95% CL LS mean difference | -69.1 -0.8 | | | |
| P-value | 0.045 | | | |

**Safety Results:** Overall, the types of adverse events that occurred during the study were consistent with those observed in patients with ESRD undergoing maintenance hemodialysis and were also generally similar between the 2 treatment groups with regard to frequency, severity, and relatedness to study drug. There were 5 subjects who died during the study: 2 in the SFP group and 3 in the placebo group. One additional subject in the placebo group died 9 days after all study visits were completed, and was not counted as an on-study death. The incidence of SAEs was similar in the SFP (18 subjects, 33.3%) and placebo (20 subjects, 40.8%) groups. Treatment-emergent AEs that led to study discontinuation were reported by relatively few subjects in the SFP (4, 7.4%) and the placebo (1, 20.0%) groups. None of the AEs that resulted in subject death, SAEs, or AEs that led to study discontinuation were considered to be related to study drug.

The incidence of infections was similar in both treatment groups (38.9% SFP and 49.0% placebo), and the incidence of intra-dialytic hypotension was also similar in both treatment groups (33.3% SFP and 40.8% placebo). There were no incidences of anaphylactoid reactions reported in either treatment group.

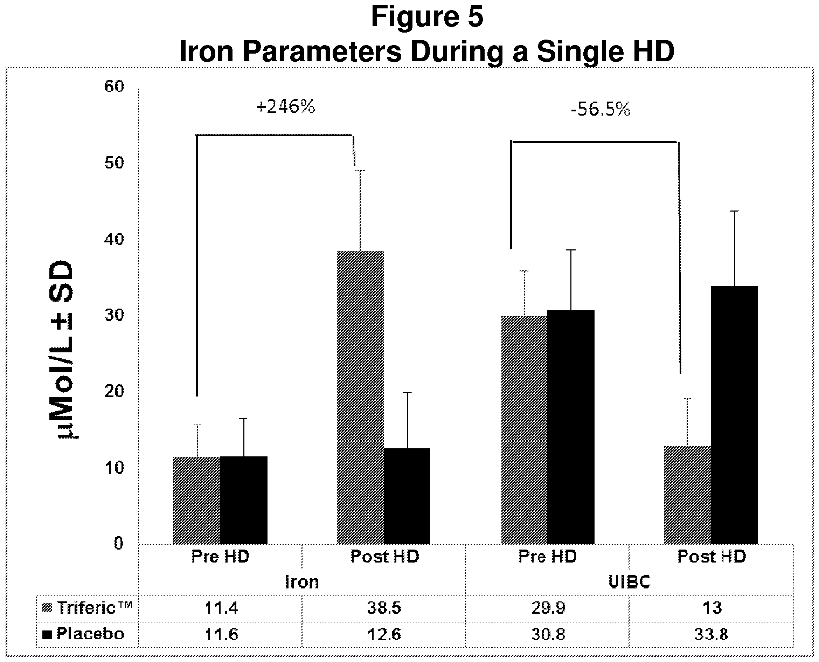

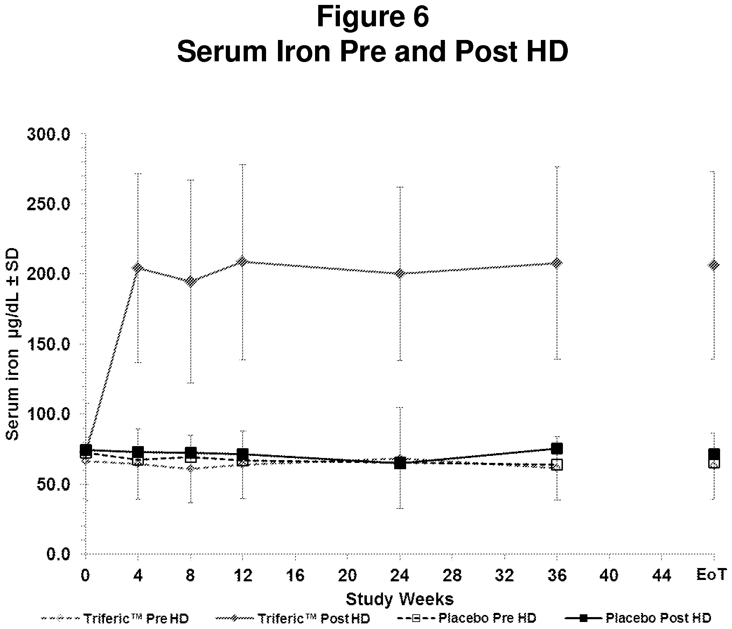

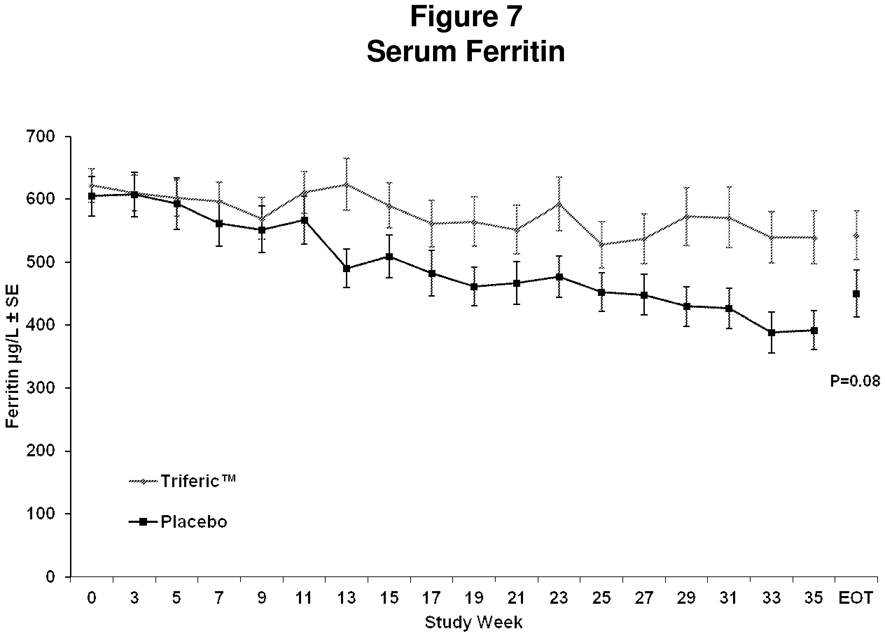

The mean pre-dialysis serum iron and TIBC values in this study were consistent with those seen in chronic inflammatory disorders such as ESRD. Mean pre-dialysis serum iron values in the SFP and placebo groups were within the normal range at most time points, but tended toward the lower range of normal. As shown in Figs. 5 and 6, mean post-dialysis iron values in the SFP group were significantly higher than corresponding values in the placebo group, indicating the delivery of iron to subjects via SFP dialysate. In subjects at risk for shift in pre-dialysis serum iron values, a greater percentage of at risk subjects in the SFP group had pre-dialysis serum iron shifts to high values, whereas placebo subjects tended to shift to low values. Mean pre-dialysis TIBC (transferrin) values were also mostly within the normal range for the placebo group across all time points, but most mean values were low for the SFP group. Serum ferritin level remained relatively stable in the SFP group while there was a significant decline in the placebo group such that there was a statistically significant difference between the two groups at the end of the study (see Fig. 7). Administration of SFP regularly 3 times a week did not result in an increase in serum ferritin level and hence did not induce tissue iron overload

### CONCLUSIONS

SFP administered via the hemodialysate was efficacious in maintaining iron sufficiency and thereby reducing the prescribed ESA dose required to maintain Hgb levels in adult ESRD patients. The safety profile of SFP was similar to that of placebo.

### Example 2

### Administration of SFP Reduces ESA Requirements in Patients with ESA Hyporesponsiveness

The subjects of PRIME Study, described above in Example 1, were divided into two strata based on the baseline ESA dose: patients receiving ≤ 13,000 U/week of ESA (stratum I) and patients receiving > 13,000 U/week ESA (stratum II; hyporesponsive to ESA). The demographics of stratum II are set out in Table 9.

**Table 9 Demographics**

| | | Strata II | |
|---|---|---|---|
| | | SFP | Placebo |
| Age (Years) | | 55.75 ± 14 | 60.3 ± 16 |
| Gender | F | 3 | 4 |
| | M | 9 | 8 |
| Race | Black | 6 | 6 |
| | White | 6 | 6 |

The percent change in the prescribed ESA dose required to maintain Hgb in the target range in the ESA hyporesponsive patients from baseline to end-of-treatment, was statistically significantly smaller in the SFP group compared to the placebo group (-8.5% vs. 65.9%, p=0.045) with a overall difference of -74.4 (see Table 10).

**Table 10**

| | Stratum I (≤ 13, 000 U/week) N = 80 | | Stratum II (> 13,000 U/week) N = 23 | |
|---|---|---|---|---|
| | Triferic | Placebo | Triferic | Placebo |
| Hgb (g/L) BL | 109.2 | 111.3 | 110.7 | 110.2 |
| Hgb (g/L) EoT | 103.7 | 103.9 | 108.5 | 104.5 |
| ESA (U/wk) BL | 7083 | 7300 | 17,483 | 16,134 |
| ESA (U/wk) EoT | 7914 | 9251 | 16,386 | 24,909 |
| LS Mean % change Mean (SE) | 7.6 (12.1) | 34.0 (12.1) | -8.5 (33.4) | 65.9 (34.8) |
| LS Mean difference | -26.4 (17.2) | | -74.4 (48.2) | |
| Mean (SE) (95% Cl) | (-60.6, 7.8) | | (-175, 26.2) | |

Hgb concentration was stable during the study, with both patient groups showing similar variability (see Table 11). The reticuloctye Hgb levels was stable in the SFP group and decreased in the placebo group (see Table 12). There was a trend towards less decrease in serum ferritin in the SFP group compared to the placebo group (see Table 13). As expected the intradialytic increase in serum iron was far greater in the SFP group compared to Placebo (see Table 14). There was a trend towards markedly lower requirement for supplemental IV iron in the SFP group compared to placebo (a secondary efficacy analysis) (see Table 15). At end-of-treatment, fewer subjects received supplemental IV iron in the SFP group (2 subjects, 8.3%) than in the placebo group (5 subjects, 20%) (see Table 16).

**Table 11**

| **Hgb (g/L)** | **SFP** | **Placebo** |
|---|---|---|
| Baseline | 111.6 ± 6.5 | 110.27 ± 7.3 |
| EoT | 108.5 ± 12.4 | 104.6 ± 9.9 |
| Change from Baseline | -3.1 ± 10.6 | -5.77 ± 15.2 |
| | p=NS | |

**Table 12**

| ***Reticulocyte Hgb (pg)*** | **SFP** | **Placebo** |
|---|---|---|
| Baseline | 32.41 ± 2.70 | 33.31 ± 2.7 |
| | 33.01 ± 2.8 | 31.04 ± 3.3 |
| Change from Baseline | 0.5 ± 2.15 | -2.3 ± 1.84 |
| | p= 0.0041 | |

**Table 13**

| **Ferritin (µg/L)** | **SFP** | **Placebo** |
|---|---|---|
| Baseline | 640 ± 118 | 524 ± 211 |
| EoT | 540 ± 150 | 315 ± 183 |
| Change from Baseline | -109 ± 150 | -209 ± 192 |
| | p= 0.1913 | |

**Table 14**

| **Serum Fe** (Average) (µMol/L) | **SFP** | | **Placebo** | |
|---|---|---|---|---|
| | Pre HD | Post HD | Pre HD | Post HD |
| EoT | 9.5 ± 2.7 | 33.6 ± 14.4 | 11.7 ± 6.1 | 14.5 ± 10.2 |

**Table 15 IV Iron Administration**

| | **N** | **Total Administered (mg)** | **Dose/Sx (mg)** |
|---|---|---|---|
| Triferic | 2 | *256* | *128 mg*/*Sx* |
| Placebo | 5 | 4200 | 840 mg/Sx |

**Table 16 Individual Subjects and dose of IV Fe in table below**

| **Subject** | **Randomization Group** | **N of doses** | **Cumulative Dose (mg)** |
|---|---|---|---|
| NIHFP01-03-012 | SFP | 2 | 131 |
| NIHFP0 1-05-003 | PLB | 14 | 2600 |
| NIHFP0 1-05-011 | PLB | 6 | 600 |
| NIHFP0 1-11-009 | PLB | 1 | 125 |
| NIHFP01-13-019 | SFP | 1 | 125 |
| NIHFP0 1-14-005 | PLB | 3 | 375 |
| NIHFP01-14-013 | PLB | 3 | 500 |

### Conclusion

The administration of SFP in the ESA hyporesponsive subjects reduced the ESA requirements compared to placebo by a mean difference of about 74%. In addition, the need for IV iron was reduced in this patient population without increasing iron stores.

## Claims

1. A dose of ferric pyrophosphate composition (SFP) for use in treating a dialysis patient that is hyporesponsive to erythropoietin stimulating agent (ESA), wherein the dose of SFP is effective to reduce the dose of ESA administered to the patient by at least 65% less than the dose of ESA required by dialysis patients that are hyporesponsive to ESA and that have not received SFP.

2. The dose of SFP for use according to claim 1 wherein the dose of SFP is administered via a parenteral route selected from the group consisting of intramuscular, subcutaneous, intravenous, intradermal, transdermal, transbuccal, sublingual, intra-peritoneal or by dialytic transfer via the hemodialysis solutions or peritoneal dialysis solutions, or orally.

3. The dose of SFP for use according to any of the preceding claims wherein the dose of SFP is administered in conjunction with other drugs such as heparin or parenteral nutrition admixtures or dialysis solutions.

4. The dose of SFP for use according to any of the preceding claims, wherein the dose of SFP increases or maintains the hemoglobin level of the patient at 9 g/dL or greater.

5. The dose of SFP for use according to any one of the preceding claims, wherein the dose of SFP is administered via hemodialysate at a dose ranging from 90 µg Fe/L dialysate to 120 µg Fe/L dialysate, preferably the SFP is administered via hemodialysate at a dose of 110 µg Fe/L dialysate.

6. The dose of SFP for use according to any one of the preceding claims, wherein the dose of SFP is administered via infusion or intravenous injection at a dose ranging from 2.4 mg to 48 mg iron per day at a rate of 0.1 to 2 mg iron per hour, or wherein the dose of SFP is administered into the circulation at a dose ranging 2.4 mg to 48 mg iron per day at a rate of 0.1 to 2 mg iron per hour.

7. The dose of SFP for use according to any one of the preceding claims, wherein the dose of ESA administered to the patient after SFP administration is at least 70% less than the dose of ESA administered prior to SFP administration.

8. The dose of SFP for use according to any of the preceding claims wherein the SFP comprises iron chelated with citrate and pyrophosphate.

9. The dose of SFP for use according to any of the preceding claims wherein the SFP comprises iron in an amount of 7% to 11% by weight, citrate in an amount of at least 14% by weight, and pyrophosphate in an amount of at least 10% by weight.

10. The dose of SFP for use according to any of the preceding claims wherein the SFP is administered via dialysate and the dose of intravenously administered iron after SFP administration is at least 10% less than the dose of intravenously administered iron prior to SFP administration.

11. The dose of SFP for use according to any of the preceding claims wherein the patient is suffering from chronic kidney disease, optionally stage III, IV or V.

12. The dose of SFP for use according to any of the preceding claims wherein the patient is suffering from anemia of inflammation and/or infection, optionally chronic infection.

13. The dose of SFP for use according to any one of the preceding claims wherein the patient is suffering from cancer, heart failure, autoimmune disease, sickle cell disease, thalassemia, blood loss, transfusion reaction, diabetes, vitamin B12 deficiency, collagen vascular disease, thrombocytopenic purpura, Celiac disease, endocrine deficiency state, hypothyroidism, Addison's disease, Crohn's Disease, systemic lupus erythematosis, rheumatoid arthritis, juvenile rheumatoid arthritis, ulcerative colitis, immune disorders, eosinophilic fasciitis, hypoimmunoglobulinemia, thrymoma/thymic carcinoma, graft vs. host disease, preleukemia, Nonhematologic syndrome, Felty syndrome, hemolytic uremic syndrome, myelodysplasic syndrome, nocturnal paroxysmal hemoglobinuria, osteomyelofibrosis, pancytopenia, pure red-cell aplasia, Schoenlein-Henoch purpura, malaria, protein starvation, menorrhagia, systemic sclerosis, liver cirrhosis, hypometabolic states, congestive heart failure, chronic infection, HIV/AIDS, tuberculosis, osteomyelitis, hepatitis B, hepatitis C, Epstein-bar virus, parvovirus, T cell leukemia virus, bacterial overgrowth syndrome, red cell membrane disorder, hereditary spherocytosis, hereditary elliptocytosis, red cell enzyme defects, hypersplenism, immune hemolysis or paroxysmal nocturnal hemoglobinuria, and/or wherein dose of SFP is administered during hemodialysis within the hemodialysate solution, and/orwherein SFP is administered during peritoneal dialysis within the peritoneal dialysis solution, and/orwherein SFP is administered with parenteral nutrition within parenteral nutrition admixture.

14. The dose of SFP for use according to any one of the preceding claims wherein SFP is administered at a therapeutically effective dose that i) increases at least one marker of iron status selected from the group consisting of serum iron, transferrin saturation, reticulocyte hemoglobin, serum ferritin, reticulocyte count, and whole blood hemoglobin and ii) decreases the dose of ESA required to achieve or maintain target hemoglobin levels, or the need for transfusion of whole blood, packed red blood cell or blood substitutes, preferably the patient is suffering from non-anemic iron deficiency and administration of the therapeutically effective dose of SFP reduces fatigue, increases physical and cognitive ability, or improves exercise tolerance in the patient.

15. The dose of SFP for use according to any one of the preceding claims wherein SFP is administered in a therapeutically effective dose that will reduce or abolish the clinical manifestations of restless leg syndrome associated with iron.

## Patentansprüche

1. Dosis einer Zusammensetzung von Tetraeisentris(pyrophosphat) (SFP) zur Verwendung beim Behandeln eines Dialysepatienten, der auf Erythropoietinstimulierende Mittel (ESA) hyporesponsiv ist, wobei die Dosis von SFP bewirkt, dass die dem Patienten verabreichte ESA-Dosis um mindestens 65 % weniger als die ESA-Dosis, die von Dialysepatienten benötigt wird, welche auf ESA hyporesponsiv sind und kein SFP erhalten haben, verringert wird.

2. SFP-Dosis zur Verwendung gemäß Anspruch 1, wobei die SFP-Dosis über einen parenteralen Weg, ausgewählt aus der Gruppe bestehend aus intramuskulär, subkutan, intravenös, intradermal, transdermal, transbuccal, sublingual, intraperitoneal oder dialytischem Transfer über die Hämodialyselösungen oder Peritonealdialyselösungen, oder oral verabreicht wird.

3. SFP-Dosis zur Verwendung gemäß einem der vorangehenden Ansprüche, wobei die SFP-Dosis in Verbindung mit anderen Arzneimitteln, wie zum Beispiel Heparin oder parenteralen Nährstoffzudosierungen oder Dialyselösungen, verabreicht wird.

4. SFP-Dosis zur Verwendung gemäß einem der vorangehenden Ansprüche, wobei die SFP-Dosis den Hämoglobinspiegel des Patienten auf 9 g/dl oder mehr anhebt oder aufrechterhält.

5. SFP-Dosis zur Verwendung gemäß einem der vorangehenden Ansprüche, wobei die SFP-Dosis über Hämodialysat in einer Dosis verabreicht wird, die von 90 µg Fe/I Dialysat bis 120 µg Fe/I Dialysat reicht, vorzugsweise das SFP über Hämodialysat in einer Dosis von 110 µg Fe/I Dialysat verabreicht wird.

6. SFP-Dosis zur Verwendung gemäß einem der vorangehenden Ansprüche, wobei die SFP-Dosis über Infusion oder intravenöse Injektion in einer Dosis im Bereich von 2,4 mg bis 48 mg Eisen pro Tag mit einer Geschwindigkeit von 0,1 bis 2 mg Eisen pro Stunde verabreicht wird oder wobei die SFP-Dosis in einer Dosis im Be- reich von 2,4 mg bis 48 mg Eisen pro Tag mit einer Geschwindigkeit von 0,1 bis 2 mg Eisen pro Stunde in den Kreislauf verabreicht wird.

7. SFP-Dosis zur Verwendung gemäß einem der vorangehenden Ansprüche, wobei die dem Patienten nach der SFP-Verabreichung verabreichte ESA-Dosis mindestens 70 % weniger als die vor der Verabreichung von SFP verabreichte ESA-Dosis beträgt.

8. SFP-Dosis zur Verwendung gemäß einem der vorangehenden Ansprüche, wobei das SFP mit Citrat und Pyrophosphat chelatisiertes Eisen umfasst.

9. SFP-Dosis zur Verwendung gemäß einem der vorangehenden Ansprüche, wobei das SFP Eisen in einer Menge von 7 bis 11 Gew.-%, Citrat in einer Menge von mindestens 14 Gew.-% und Pyrophosphat in einer Menge von mindestens 10 Gew.-% umfasst.

10. SFP-Dosis zur Verwendung gemäß einem der vorangehenden Ansprüche, wobei das SFP über Dialysat verabreicht wird und die Dosis von intravenös verabreichtem Eisen nach Verabreichung von SFP mindestens 10 % weniger als die Dosis von intravenös verabreichtem Eisen vor der Verabreichung von SFP beträgt.

11. SFP-Dosis zur Verwendung gemäß einem der vorangehenden Ansprüche, wobei der Patient an einer chronischen Nierenerkrankung, wahlweise Stadium III, IV oder V, leidet.

12. SFP-Dosis zur Verwendung gemäß einem der vorangehenden Ansprüche, wobei der Patient an Anämie bei Entzündung und/oder Infektion, wahlweiser chronischer Infektion, leidet.

13. SFP-Dosis zur Verwendung gemäß einem der vorangehenden Ansprüche, wobei der Patient an Krebs, Herzversagen, Autoimmunkrankheit, Sichelzellenanämie, Thalassämie, Blutverlust, Transfusionsreaktion, Diabetes, Vitamin-B12-Mangel, Kollagen-Gefäßerkrankung, thrombozytopenischer Purpura, Zöliakie, endokrinem Mangelzustand, Hypothyreose, Addison-Krankheit, Morbus Crohn, systemischem-Lupus erythematodes, rheumatoider Arthritis, juveniler rheumatoider Arthritis, Colitis ulcerosa, Immunstörungen, eosinophiler Fasziitis, Hypoimmunglobulinämie, Thymom/Thymuskarzinom, Graft-versus-Host-Krankheit, Präleukämie, nichthämatologischem Syndrom, Felty-Syndrom, hämolytisch-urämischem Syndrom, myelodysplastischem Syndrom, nächtlicher paroxysmaler Hämoglobinurie, Osteomyelofibrose, Panzytopenie, isolierter aplastischer Anämie, Purpura Schoenlein-Henoch, Malaria, Proteinmangel, Menorrhagie, systemischer Sklerose, Leberzirrhose, hypometabolischen Zuständen, kongestiver Herzinsuffizienz, chronischer Infektion, HIV/AIDS, Tuberkulose, Osteomyelitis, Hepatitis B, Hepatitis C, Epstein-Barr-Virus, Parvovirus, T-Zell-Leukämievirus, bakteriellem Überwucherungssyndrom, Erythrozytenmembran-Störung, hereditärer Sphärozytose, hereditärer Elliptozytose, Erythrozyten-Enzymdefekten, Hypersplenismus, Immunhämolyse oder paroxysmaler nächtlicher Hämoglobinurie leidet und/oder wobei die SFP-Dosis während der Hämodialyse in der Hämodialysatlösung verabreicht wird und/oder wobei SFP während der Peritonealdialyse in der Peritonealdialysatlösung verabreicht wird und/oder wobei SFP mit der parenteralen Ernährung in einer parenteralen Nährstoffzudosierung verabreicht wird.

14. SFP-Dosis zur Verwendung gemäß einem der vorangehenden Ansprüche, wobei SFP in einer therapeutisch wirksamen Dosis verabreicht wird, die i) mindestens ein Markierungszeichen des Eisenstatus, ausgewählt aus der Gruppe bestehend aus Serumeisen, Transferrinsättigung, Retikulozytenhämoglobin, Serumferritin, Retikulozytenzahl und Hämoglobin im Vollblut, erhöht und ii) die ESA-Dosis, die erforderlich ist, um Ziel-Hämoglobin-Spiegel zu erreichen oder aufrechtzuerhalten, oder den Bedarf nach einer Transfusion von Vollblut, Erythrozytenkonzentraten oder Blutersatzstoffen verringert, bevorzugt der Patient an nicht-anämischem Eisenmangel leidet und die Verabreichung der therapeutisch wirksamen Dosis von SFP die Müdigkeit verringert, die körperliche und kognitive Fähigkeit erhöht oder die Belastungstoleranz bei dem Patienten verbessert.

15. SFP-Dosis zur Verwendung gemäß einem der vorangehenden Ansprüche, wobei SFP in einer therapeutisch wirksamen Dosis, welche die klinischen Manifestationen des mit Eisen verbundenen Restless-Leg-Syndroms verringern oder aufheben wird, verabreicht wird.

## Revendications

1. Dose de composition de pyrophosphate ferrique (SFP) pour l'utilisation dans le traitement d'un patient sous dialyse qui est hyposensible à l'agent de stimulation de l'érythropoïétine (ESA), où la dose de SFP est efficace pour réduire la dose d'ESA administrée au patient au moins de 65 % inférieure à la dose d'ESA requise par les patients sous dialyse qui sont hyposensibles à l'ESA et qui n'ont pas reçu de SFP.

2. Dose de SFP pour l'utilisation selon la revendication 1 dans laquelle la dose de SFP est administrée par l'intermédiaire de la voie parentérale sélectionnée dans le groupe constitué du transfert intramusculaire, sous-cutané, intraveineux, intradermique, transdermique, transbuccal, sublingual, intrapéritonéal ou par transfert dialytique par l'intermédiaire de solutions d'hémodialyse ou de solutions de dialyse péritonéales, ou par voie orale.

3. Dose de SFP pour l'utilisation selon l'une quelconque des revendications précédentes dans laquelle la dose de SFP est administrée en conjonction avec d'autres médicaments tels que l'héparine ou des mélanges par admixtion de nutrition parentérale ou des solutions de dialyse.

4. Dose de SFP pour l'utilisation selon l'une quelconque des revendications précédentes, dans laquelle la dose de SFP accroît ou maintient le niveau d'hémoglobine du patient à 9 g/dl ou plus.

5. Dose de SFP pour l'utilisation selon l'une quelconque des revendications précédentes, dans laquelle la dose de SFP est administrée par l'intermédiaire d'hémodialysat à une dose s'étendant de 90 µg Fe/l de dialysat à 120 µg Fe/l de dialysat, préférablement le SFP est administré par l'intermédiaire d'hémodialysat à une dose de 110 µg Fe/l de dialysat.

6. Dose de SFP pour l'utilisation selon l'une quelconque des revendications précédentes, la dose de SFP étant administrée par l'intermédiaire de la perfusion ou injection intraveineuse à une dose s'étendant de 2,4 mg à 48 mg de fer par jour à une vitesse de 0,1 à 2 mg de fer par heure, ou la dose de SFP étant administrée dans la circulation à une dose s'étendant de 2,4 mg à 48 mg de fer par jour à une vitesse de 0,1 à 2 mg de fer par heure.

7. Dose de SFP pour l'utilisation selon l'une quelconque des revendications précédentes, la dose d'ESA administrée au patient après l'administration de SFP étant au moins de 70 % inférieure à la dose d'ESA administrée avant l'administration de SFP.

8. Dose de SFP pour l'utilisation selon l'une quelconque des revendications précédentes, le SFP comprenant du fer chélaté avec du citrate et du pyrophosphate.

9. Dose de SFP pour l'utilisation selon l'une quelconque des revendications précédentes, le SFP comprenant du fer en une quantité de 7 % à 11 % en poids, du citrate en une quantité d'au moins 14 % en poids, et du pyrophosphate en une quantité d'au moins 10 % en poids.

10. Dose de SFP pour l'utilisation selon l'une quelconque des revendications précédentes, le SFP étant administré par l'intermédiaire de dialysat et la dose de fer administrée par voie intraveineuse après l'administration de SFP étant au moins de 10 % inférieure à la dose de fer administrée par voie intraveineuse avant l'administration de SFP.

11. Dose de SFP pour l'utilisation selon l'une quelconque des revendications précédentes, le patient souffrant de maladie rénale chronique, éventuellement au stade III, IV ou V.

12. Dose de SFP pour l'utilisation selon l'une quelconque des revendications précédentes, le patient souffrant d'anémie ou d'inflammation et/ou d'infection, éventuellement d'infection chronique.

13. Dose de SFP pour l'utilisation selon l'une quelconque des revendications précédentes, le patient souffrant de cancer, d'insuffisance cardiaque, de maladie auto-immune, de drépanocytose, de thalassémie, de perte de sang, de réaction à la transfusion, de diabète, de carence en vitamine B12, de maladie vasculaire liée au collagène, de purpura thrombocytopénique, de la maladie coeliaque, d'état d'insuffisance endocrinienne, d'hypothyroïdie, de la maladie d'Addison, de la maladie de Crohn, du lupus érythémateux systémique, de l'arthrite rhumatoïde, de l'arthrite rhumatoïde juvénile, de la rectocolite hémorragique, de troubles immuns, de fasciite éosinophile, d'hypoimmunoglobulinémie, de thymome/carcinome thymique, de la maladie du greffon *versus* l'hôte, de la préleucémie, du syndrome non hématologique, du syndrome de Felty, du syndrome hémolytique urémique, du syndrome de myélodysplasie, d'hémoglobinurie nocturne paroxysmique, d'ostéomyélofibrose, de pancytopénie, d'aplasie pure des hématies, de purpura de Schoenlein-Henoch, de paludisme, de carence en protéine, de ménorragie, de sclérose systémique, de cirrhose hépatique, d'états hypométaboliques, d'insuffisance cardiaque congestive, d'infection chronique, du VIH/SIDA, de la tuberculose, de l'ostéomyélite, de l'hépatite B, de l'hépatite C, du virus d'Epstein-bar, du parvovirus, du virus de la leucémie à cellules T, du syndrome de surdéveloppement bactérien, de trouble des membranes des hématies, de la sphérocytose héréditaire, de l'elliptocytose héréditaire, de déficits enzymatiques érythrocytaires, d'hypersplénisme, d'hémolyse immune ou d'hémoglobinurie nocturne paroxysmique, et/ou la dose de SFP étant administrée durant l'hémodialyse dans la solution d'hémodialysat, et/ou le SFP étant administré durant la dialyse péritonéale à l'intérieur de la solution de dialyse péritonéale, et/ou le SFP étant administré avec nutrition parentérale à l'intérieur d'un mélange par admixtion de nutrition parentérale.

14. Dose de SFP pour l'utilisation selon l'une quelconque des revendications précédentes, le SFP étant administré à une dose thérapeutiquement efficace qui i) augmente au moins un marqueur d'état du fer sélectionné dans le groupe constitué du fer sérique, de la saturation transferrine, de l'hémoglobine de réticulocyte, de la ferritine sérique, de la numération des réticulocytes, et de l'hémoglobine de sang total et ii) réduit la dose d'ESA requise pour atteindre ou maintenir les niveaux cibles d'hémoglobine, ou le besoin de transfusion de sang total, de globules rouges de culot globulaire ou de substituts du sang, préférablement le patient souffre de carence non anémique en fer et l'administration de la dose thérapeutiquement efficace de SFP réduit la fatigue, augmente les capacités physiques et cognitives, ou améliore la tolérance à l'exercice chez le patient.

15. Dose de SFP pour l'utilisation selon l'une quelconque des revendications précédentes, le SFP étant administré en une dose thérapeutiquement efficace qui réduira ou abolira les manifestations cliniques du syndrome des jambes sans repos associé au fer.
